# EUROPÄISCHE PATENTSCHRIFT

(11) **EP 1 173 550 B1**
(45) Veröffentlichungstag und Bekanntmachung des Hinweises auf die Patenterteilung: **26.07.2006**
(21) Anmeldenummer: 00936709.5
(22) Anmeldetag: 04.05.2000
(51) Int. Cl.: C12N 5/10, A61K 35/14, A61K 48/00

(54) **VERFAHREN ZUR REDUZIERUNG VON SPEZIFISCHEN IMMUNREAKTIONEN**
METHOD FOR DIMINISHING SPECIFIC IMMUNE REACTIONS
PROCEDE POUR REDUIRE DES IMMUNOREACTIONS SPECIFIQUES

(30) Priorität: 04.05.1999 DE 19920412; 18.09.1999 EP 99118518; 18.09.1999 DE 19944858
(43) Veröffentlichungstag der Anmeldung: 23.01.2002
(73) Patentinhaber: Genethor GmbH, 13125 Berlin (DE)
(72) Erfinder: SHERIFF, Ahmed, D-10713 Berlin (DE); GEBAUER, Frank, D-10963 Berlin (DE)
(74) Vertreter: Meyers, Hans-Wilhelm
(86) Internationale Anmeldenummer: PCT/EP2000/003984
(87) Internationale Veröffentlichungsnummer: WO 2000/066715

(56) Entgegenhaltungen:
- WO-A-98/29124
- WO-A-99/25812
- WO-A-99/50394
- DATABASE BIOSIS [Online] BIOSCIENCES INFORMATION SERVICE, PHILADELPHIA, PA, US; 1992 SHIOTA Y ET AL: "INDUCTION OF UP-MODULATION OF HOMING RECEPTORS IN CLONED HEMOPOIETIC PROGENITORS BY GROWTH FACTORS" Database accession no. PREV199293119387 XP002142748 & BONE MARROW TRANSPLANTATION, Bd. 9, Nr. 2, 1992, Seiten 123-127, ISSN: 0268-3369
- L. LU ET AL.: "TRANSDUCTION OF DENDRITIC CELLS WITH ADENOVIRAL VECTORS ENCODING CTLA4-Ig MARKEDLY REDUCES THEIR ALLOSTIMULATORY ACTIVITY." TRANSPLANTATION PROCEEDINGS, Bd. 31, Nr. 1/2, März 1999 (1999-03), Seite 797 XP000915484 NEW YORK, N.Y., US
- R.W. O'ROURKE ET AL.: "A DENDRITIC CELL LINE GENETICALLY MODIFIED TO EXPRESS CTLA4-Ig AS A MEANS TO PROLONG ISLET ALLOGRAFT SURVIVAL." TRANSPLANTATION, Bd. 69, Nr. 7, 15. April 2000 (2000-04-15), Seiten 1440-1446, XP000915486 BALTIMORE, US
- F. FU ET AL.: "COSTIMULATORY MOLECULE-DEFICIENT DENDRITIC CELL PROGENITORS (MHC CLASS II+, CD80dim, CD86-) PROLONG CARDIAC ALLOGRAFT SURVIVAL IN NONIMMUNOSUPPRESSED RECIPIENTS." TRANSPLANTATION, Bd. 62, Nr. 5, 15. September 1996 (1996-09-15), Seiten 659-665, XP000929113 BALTIMORE, US

## Beschreibung

Gegenstand der vorliegenden Erfindung sind Antigen präsentierende Zellen, Verfahren zur Herstellung Antigen präsentierender Zellen, Arzneimittel enthaltend Antigen präsentierende Zellen sowie Verwendung der Antigen präsentierenden Zellen

### Beschreibung

Seit knapp 10 Jahren werden für verschiedene Krankheiten und in Tiermodellen gentherapeutische Verfahren entwickelt und angewendet. Bisher sind sie noch nicht in die Routine überführt. Meist handelt es sich dabei um die Behandlung von genetisch bedingten, schweren Erkrankungen, für die andere Therapien nicht zur Verfügung stehen. Weiterhin werden Gentherapien zur Behandlung von schweren und ebenfalls nicht therapierbaren Krebserkrankungen eingesetzt.

Wenig Aufmerksamkeit hat die experimentelle Medizin bisher der Behandlung von Allergien und Autoimmunerkrankungen mittels gentechnischer Methoden geschenkt.

### Allgemeines zu Allergien

Allergien verursachen beträchtliche Kosten im Gesundheitswesen der Industrieländer. Immerhin sind schätzungsweise mindestens 20% der Bevölkerung gegen irgendeine Substanz allergisch. Die meisten Betroffenen leiden an allergischer Rhinitis, zu der insbesondere der Heuschnupfen zählt, oder an Bronchialasthma: Sie niesen oder ringen nach Luft, nachdem sie bestimmte Pollen oder andere im allgemeinen harmlose Substanzen eingeatmet haben. Viele Kinder und einige Erwachsene reagieren auch allergisch auf Nahrungsmittel. Andere erleiden Hautausschläge oder sogar einen allergischen Schock, nachdem sie Medikamente wie Penizillin erhalten haben. Bei wieder anderen rufen Bienenstiche starke lokale Schwellungen oder schwere systemische - den gesamten Organismus erfassende - Störungen hervor. Im Extremfall können allergische Anfälle sogar zum Tod führen. Allein die unmittelbare medizinische Versorgung von Asthmapatienten hat in den USA 1990 schätzungsweise 3,6 Milliarden Dollar verschlungen und damit rund ein Prozent aller Kosten im Gesundheitswesen ausgemacht.

Mittlerweile ist bekannt, dass einige der zellulären und molekularen Wechselwirkungen bei allergischen Reaktionen oft ähnlich ablaufen, unabhängig davon, auf welche Substanzen der einzelne anspricht und welche Symptome er entwickelt. Gewisse Begleiterscheinungen der Allergien treten normalerweise ausschließlich auf, wenn das Immunsystem Parasiten bekämpft. So reagiert der Körper auf Schmarotzer ebenso wie auf Allergene mit der massiven Produktion von Molekülen, die als Immunglobulin-E-Antikörper (IgE) bezeichnet werden. Die Produktion dieser Antikörper wird durch T-Helfer-Zellen induziert, die wiederum von antigen-präsentierenden Zellen aktiviert werden.

### Sensibilisierung

Unterschiedliche Allergene rufen unter anderem deswegen verschiedenartige Symptome hervor, weil sie mit dem Immunsystem in verschiedenen Körperregionen in Berührung kommen. In den oberen Luftwegen erzeugt die fehlgeleitete Immunreaktion Niesen und eine verstopfte Nase. In den unteren Luftwegen können dagegen die Bronchien sich verengen und verschleimen, so dass typische asthmatische Symptome auftreten. Entsprechend rufen Immunaktivitäten in den Geweben des Magen-Darm-Traktes Übelkeit, Bauchkrämpfe, Durchfall oder Erbrechen hervor.

Schließlich vermag ein Allergen, dass auf irgendeinem Weg ins Blut gelangt, eine Anaphylaxe auszulösen: eine allergische Reaktion in weit von seiner Eintrittsstelle entfernten Körperregionen. Schwere anaphylaktische Schocks können alle normalen Körperfunktionen durcheinanderbringen und tödlich enden.

Auch wenn sich die allergischen Reaktionen unterschiedlich äußern, werden sie doch stets durch den gleichen Mechanismus in Gang gesetzt: die Sensibilisierung. Dazu kann bereits der einmalige Kontakt mit einem Allergen, typischerweise einem Eiweißstoff, genügen. In den Luftwegen oder anderen Geweben trifft die allergieauslösende Substanz auf sogenannte Fresszellen oder Makrophagen. Diese verschlingen den Fremdstoff, zerstückeln ihn und präsentieren die Fragmente auf der Zelloberfläche mit MHC II-Molekülen. Im weiteren Verlauf erkennen einige T-Helfer-Lymphozyten die dargebotenen Bruchstücke und binden daran. Die T-Helfer-Lymphozyten werden von den Makrophagen aktiviert und aktivieren dann ihrerseits einige B-Lymphozyten, die gleichfalls das Allergen erkennen. Die B-Zellen reifen dann zu Antikörper produzierenden Plasmazellen aus. Zunächst sind dies Antikörper vom sogenannten IgM-Typ; ab einem bestimmten Zeitpunkt schalten die Plasmazellen jedoch auf IgE-Antikörper um.

Bis die Antikörper hergestellt sind, können Tage oder Wochen vergehen, und das Allergen, welches ihre Produktion in Gang gesetzt hat, ist dann womöglich schon lange verschwunden. Nicht so die IgE-Moleküle. Mit ihrer Fc-Region heften sie sich an IgE-Rezeptoren zweier unterschiedlicher Klassen von Immunsystemzellen. Bei der einen handelt es sich um Mastzellen, die sich im Körpergewebe für gewöhnlich in der Nähe von Blutgefäßen und Epithelzellen ansiedeln. Über das Epithel besteht Kontakt mit der Außenwelt (darunter fällt auch das Epithel der Atemwege und des Magen-Darm-Traktes). IgE-Antikörper binden außerdem an basophile Granulozyten (Basophile). Diese Zellen zirkulieren allerdings im Blutstrom.

Hat die Produktion der IgEs einmal begonnen, hält sie offenbar Monate, ja manchmal sogar Jahre an. Folglich besetzen sie unablässig IgE-Rezeptoren auf Mastzellen und Basophilen - bereit beim nächsten Allergenkontakt augenblicklich in Aktion zu treten.

### Akute Symptome

Während also die erste Begegnung mit einem Allergen selbst bei Personen, die sich später als Allergiker entpuppen, keine Symptome hervorruft, leitet die Zweitexposition ein Stadium der Überempfindlichkeitsreaktion ein, welches auch äußerlich in Erscheinung tritt. Innerhalb von Sekunden nach dem Kontakt mit menschlichem Gewebe bindet der allergieauslösende Stoff an die IgEs der Mastzellen. Heftet er sich dabei an zwei oder mehr IgE-Moleküle zugleich, bildet er eine Brücke zwischen ihnen. Solche Quervernetzungen lassen die betroffenen IgE-Rezeptoren dichter zusammenrücken, und dies aktiviert die Zelle, so dass sie hochwirksame Substanzen ausschüttet, die auf direktem Wege allergische Symptome erzeugen. (Die Freisetzung kann auch auf andere Arten hervorgerufen werden; von allergischen Reaktionen spricht man nur, wenn IgEs beteiligt sind). Die wichtigste dieser Substanzen ist Histamin. Es kann sowohl die Schleimbildung in den Epithelien anregen und so zur Verstopfung der Luftwege beitragen als auch die glatte Muskulatur, die wie ein elastisches Band Bronchien und Därme umschlingt, kontrahieren lassen. Ferner vermag es die feinen Blutgefäße zu weiten und durchlässiger zu machen, so dass Flüssigkeit ins Gewebe sickern kann. Rötungen und Schwellungen sind die Folge. Betreffen diese Gefäßveränderungen große Teile des Körpers, können sie ein tödliches Kreislaufversagen auslösen: Bei einem solchen Schock fällt der Blutdruck jäh so stark ab, dass die Sauerstoffversorgung von Herz und Gehirn nicht mehr gewährleistet ist.

Die zweite Gruppe von Mediatoren besteht hauptsächlich aus Prostaglandinen und Leukotrienen. Sie werden erst erzeugt, nachdem die Allergenmoleküle sich an die IgEs auf den Zellen angelagert haben. Wie Histamin verengen sie die Bronchien und erweitern die Blutgefäße. Ihre Wirkung hält allerdings länger an.

Zusätzlich stoßen stimulierte Mastzellen eine Vielzahl potentiell toxischer Enzyme aus. Offenbar setzen sie ferner Cytokine frei, die die Aktivitäten anderer Immunzellen regulieren.

### Behandlung: 1. Die allergische Rhinitis

Antihistaminika erweisen sich in der Regel als wirksam und dienen immer noch als Standardtherapie. Die neuesten Varianten können die Blut-Hirn-Schranke nicht mehr ohne weiteres passieren und machen die Patienten nicht mehr müde. Wenn bei einer schweren Entzündung Antihistaminika wirkungslos bleiben, helfen oft inhalierbare Corticosteroide, die gewöhnlich zur Linderung der chronischen Entzündung bei Asthma verschrieben werden.

In schweren Fällen kann die schon im Jahre 1911 eingeführte Immuntherapie oder Hyposensibilisierung (auch als Allergiespritzen oder Desensibilisierung bekannt) auf lange Sicht Erleichterung verschaffen. Bei dieser Behandlung injizieren Ärzte den Patienten steigende Dosen des Allergens, auf das diese empfindlich reagieren. In allen Fällen ist die Dosis ausschlaggebend: Zu wenig Allergen verleiht keine Toleranz. Außerdem ist der Schutz selten vollständig.

### 2. Asthma

Bronchodilatatoren sind die meistverwendeten Arzneimittel bei Asthma. Sie lindern die durch Histamin und andere Bronchokonstriktoren hervorgerufenen Symptome sehr schnell, beeinflussen die zugrundeliegende Entzündung jedoch wahrscheinlich nicht. Außerdem kann ihr übermäßiger Gebrauch eine Gegenreaktion des Körpers hervorrufen, so dass nach Abklingen ihrer Wirkung der Atemstrom stärker behindert ist als zuvor. Zusätzlich kommen die unter 1. aufgezählten Methoden zur Anwendung.

### 3. Anaphylaktische Reaktionen

Insektenstiche rufen bei manchen Menschen Anaphylaxien aus. In schweren Fällen führen diese zum Tod durch z.B. Kreislaufversagen oder Ersticken. Jede schwere Anaphylaxie - gleich ob sie zum ersten oder fünfzehnten Mal auftritt - ist ein Notfall, bei dem zunächst versucht werden muss, die bedrohlichsten Symptome zu beherrschen. Meist geschieht das durch Injektion von Adrenalin, welches die Freisetzung der Mediatoren hemmt, die Luftwege öffnet und der Erweiterung der Blutgefäße entgegenwirkt. Man kann vorbeugend durch eine Immunisierung mit dem Gift des gesundheitsbedrohenden Insekts agieren.

### Neue Ansätze

In der Erprobung im Tiermodell befindet sich die auf DNA basierende Immunisierung mit einem Allergen (Der p 5) der Milbe *Dermatophagoides pteronyssinus.* Diese Immunisierung resultiert in einer Produktion von IgG, aber nicht IgE, und resultiert in einer 90%tigen Reduktion der Mengen an spezifischem IgE, welche durch klassische Sensibilisierung mit Der p 5 und Alaun als Adjuvant oder allergen-induzierte Rhinitis hervorgerufen wurden.

Eine weitere neue Strategie ist die Verwendung von humanisierten monoklonalen Anti-IgE-Antikörpern gegen die FcεRI-Binderegion für IgE. Dadurch wird die Bindung von IgE an den IgE-Rezeptor verhindert, so dass keine Mediatoren der allergischen Reaktion von Mastzellen oder Basophilen ausgeschüttet werden können. Diese Strategie hat in klinischen Studien bei Patienten mit allergischer Rhinitis und allergischem Asthma gezeigt, dass diese Antikörper gut toleriert werden und die allergischen Reaktionen reduzieren.

(siehe auch L.M. Lichtenstein: "Allergie und Immunsystem"; in Spektrum der Wissenschaft Spezial: Das Immunsystem; 1994; 74-83; S-K Huang, K-Y Chua und K-H Hsieh: Allergen gene transfer. Current Opinion in Immunology 1997; 800-804; C. Heusser und P. Jardieu: Therapeutic potential of anti-IgE antibodies. Current Opinion in Immunology 1997; 805-814).

### Allgemeines zu Transplantationen

Die Transplantation von Geweben, um kranke Organe zu ersetzen, ist heute eine wichtige medizinische Therapie. In den meisten Fällen stellt eine Reaktion des anpassungsfähigen Immunsystems gegen das Transplantat die größte Bedrohung für eine erfolgreiche Behandlung dar. Reaktionen des anpassungsfähigen Immunsystems werden von Antigen-präsentierenden Zellen durch Aktivierung von T-Helfer-Lymphozyten induziert. Bei der Transfusionen von Blut, welches das erste und am häufigsten verwendete Transplantat ist, müssen die ABO und Rh Blutgruppenantigene abgeglichen werden, damit die schnelle Zerstörung unpassender Erythrozyten vermieden wird. Bei anderen Geweben müssen die sehr polymorphen Haupthistokompatibilätskomplexe (MHC) aufeinander abgeglichen werden, da diese fast immer die Immunreaktion auslösen. Leider ist der perfekte Abgleich der MHCs außer bei Verwandten fast unmöglich.

Obwohl die Immunreaktion Organtransplantationen schwierig macht, gibt es wenige Alternativen bei Organausfällen. Die Verwendung von potenten immunsuppressiven Drogen, besonders Cyclosporin A und FK-506, die die Aktivierung von T-Zellen verhindern, macht Organtransplantationen erfolgreich. Trotzdem laufen hier einige Probleme auf, da das Leiden, welches das eigene Organ zerstört hat, auch das fremde Organ zerstört. Außerdem steigt durch die Unterdrückung des Immunsystems, das Risiko an Krebs oder Infektionen zu erkranken. Zudem ist die Prozedur sehr kostspielig (Janeway and Travers 1997).

### Allgemeines zu Autoimmunerkrankungen

Autoimmunerkrankungen gehören zu den chronischen Erkrankungen. Zu ihnen zählen Rheuma in verschiedensten klinischen Ausprägungen, Diabetes, Multiple Sklerose, bestimmte Formen von Herzmuskelentzündungen und von Schilddrüsenerkrankungen. Die Reihe von Autoimmunerkrankungen ließe sich beliebig fortsetzen, wobei ca. 90% allerdings epidemiologisch nur einen kleinen Prozentsatz ausmachen.

Die klinischen Verläufe von Autoimmunerkrankungen selbst bei ein- und derselben Diagnose können sehr unterschiedlich sein. Z. T. werden schubartige Krankheitsverläufe beobachtet. Entsprechend werden die Behandlungen vom Arzt individuell gestaltet.

Einige der Autoimmunerkrankungen sind antikörpervermittelt. Darunter wird in der Immunologie verstanden, dass der Organismus aus meist nicht bekannten Ursachen Antikörper produziert, welche sich gegen körpereigene zelluläre Strukturen (Autoantigene) richten. Sind diese Antikörper einmal gebildet, lösen sie durch ihre Interaktion und Bindung an die jeweiligen organ- oder zellspezifischen Strukturen eine zell- und gewebszerstörende Reaktion des Immunsystems aus. Letztendlich führt diese dann zu dem klinischen Bild der Erkrankung (Steinman 1994).

Beispiel hierfür ist die Schilddrüsenerkrankung Morbus Basedow, aber auch eine Form der Herzmuskelentzündung, die zur Dilatativen Cardiomyopathie führt. In einigen Fällen sind sowohl die Targets, gegen die sich die Autoantikörper richten, als auch die Autoantikörper selbst, genau charakterisiert.

WO-A-96/18736 offenbart ein Verfahren und ein Reagenz zur Behandlung von arthritischen Bedingungen, Induktion von Toleranz bei Verpflanzungen und Umkehrung von Immunreaktionen. Dabei wird die Möglichkeit, die Expression von B7-1, B7-2 und B7-3 durch Ribozyme oder Antisense-RNA zu unterdrücken, theoretisch erörtert. Die Unterdrückung der B7-Mengen durch ein B7-Ribozym und B7-Antisense-RNA werden zwar nicht durch Experimente verifiziert, aber eine reine Unterdrückung von B7 würde im besten Falle eine allgemeine Reduzierung der gesamten Reaktionen des anpassungsfähigen Immunsystems bewirken.

Die WO-A-95/32734 beschreibt die Verhinderung co-stimulatorischer Aktivitäten von antigen-präsentierenden Zellen, indem FcγRII-Rezeptoren durch aggregierte IgG-Moleküle vernetzt werden. Eine Unterdrückung von T-Zell-vermittelten Krankheiten soll erreicht werden, indem gleichzeitig die FcγRII-Rezeptoren vernetzt und spezifische Antigene verabreicht werden.

Die WO-A-98/29124 betrifft injizierte Antisense-Oligonukleotide, mit denen die B7-Mengen reduziert werden sollen. Damit kann die Expression von B7 höchstens unspezifisch in allen Zellen behindert werden.

WO-A-97/44450 offenbart die Erhöhung oder Unterdrückung von Prozessen, die auf sequenzspezifischen Interaktionen von Nukleinsäuren mit der Zellmaschinerie beruhen. Insbesondere können Genpromotor-unterdrückende Nukleinsäuren eingesetzt werden, die mit Genpromotormotiven für die Erhöhung oder Unterdrückung der Transkription eines Zielgens interagieren. Im Detail werden mit einer Utron-Nukleinsäure, dem TSU, die Expression von MHCs der Klassen I und II, sowie von ICAM-1, B7-1, B7-2 und FcγR behindert. Ein solcher Eingriff könnte insbesondere für Organtransplantate von Nutzen sein, bei denen die Expression von MHC-Molekülen die entscheidenden Abstoßungsreaktionen hervorruft. Utron-Nukleinsäuren stammen aus den 3'-untranslatierten Regionen von mRNAs. Diese 3'-untranslatierten Regionen werden so genannt, wenn sie *in vivo* Aktivitäten entfalten, die zelluläre Prozesse durch sequenzspezifische Interaktionen mit einem Teil der zellulären Maschinerie stimulieren oder inhibieren. Bei der zellulären Maschinerie handelt es sich gemäß WO -A-97/44450 um Promotoren. Es wird dort zwar über die Möglichkeit der Unterdrückung der B7-Expression (1 und 2) mit gentechnischen Methoden spekuliert, aber eine solche Inhibition kann mit dem aufgezeigten Mittel nur in Zusammenhang mit der Inhibition weiterer Gene (MHCs der Klassen I und II, sowie von ICAM-1 und FcγR) geschehen. Eine solch weitreichende Inhibition ist kontraproduktiv, da man auf eine hohe MHC-Expression angewiesen ist. Außerdem ist eine Regulation auf der Promotorebene nachteilig, da keine Möglichkeit besteht, um hinreichend spezifisch zu wirken.

WO-A-99/50394 offenbart teilungsfähige aus Monozyten gewonnene Zellen. Es werden keine Monozyten beschrieben, die vorwiegend vordefinierte Antigene präsentieren. Es wird kein Gentransfer vorgesehen, der für eine verminderte Anzahl von CD80, CD86, CD40 an der Zelloberfläche sorgt. Vielmehr wird deren verminderte Präsenz durch die Behandlung mit z. B. physikalischen Methoden wie UV-Licht erreicht, wodurch auch die beschriebenen anderen Charakteristika hervorgerufen werden.

Das der Erfindung zu Grunde liegende Problem besteht unter anderem darin, gentechnologische, therapeutisch nutzbare Produkte für die Reduktion von spezifischen Immunreaktionen zur Verfügung zustellen, bei denen Targets (Antigene, Autoantigene) und Antikörper, Autoantikörper bekannt sind.

Gelöst wird dieses Problem durch die erfindungsgemäße antigen-präsentierende Zelle, die durch Transformation einer antigen-präsentierenden Zelle mit nukleinsäurehaltigem Material erhältlich ist, wobei das nukleinsäurehaltige Material eine erhöhte Expression eines vorher bestimmten Antigens sowie eine Suppression von Funktionen der B7- und/oder CD40-Rezeptoren bewirkt.

Vorteilhaft an der antigen-präsentierenden Zelle gemäß der Erfindung ist, dass man die Zellen vermehren und z. B. Makrophagen und dendritischen Zellen differenzieren kann. Durch Erhalt der Teilungsfähigkeit werden diese Zellen im Grunde erst für den Gentransfer durch die gängigen nicht replikationsfähigen Retroviren geeignet, da diese erst bei der Teilung ins Genom eingebaut werden.
Figur 1 zeigt ein Schema zur Funktion der Genmanipulation auf zellulärer Ebene.
   a) normaler Mechanismus der T-Helfer-Zell-Aktivierung über Monozyten
   b) Mechanismus der Monozyten nach Genmanipulation. Ausbleiben der T-Helfer-Zell-Aktivierung nach Genmanipulation der Monozyten.
Figur 2 zeigt eine "Normale" Immunreaktion zur Antikörperbildung, Antigen-präsentierende Monozyten als Induktoren der Antikörperproduktion. Monozyten nehmen als fremd erkannte Moleküle auf und bringen sie auf die Zelloberfläche (Antigenpräsentation). Gemeinsam mit einem Oberflächenmolekül (B7) wird das Antigen den T-Helferzellen präsentiert. Das präsentierte Antigen wird von T-Helfer-Lymphozyten erkannt, die ihrerseits B-Zellen zur Antikörperproduktion anregen. Das Schema ist stark vereinfacht.
Figur 3 zeigt schematisch die Funktion der Genmanipulation auf molekularer Ebene: der Co-stimulierende Rezeptor B7 wird inaktiviert.
Figur 4 (I) zeigt die Funktion der Genmanipulation auf molekularer Ebene: das Autoantigen wird von den B7-inhibierten antigenpräsentierenden Zellen synthetisiert
Figur 4 (II) zeigt einen Vergleich der Antigenpräsentation von "normalen" und gentechnisch veränderten Monozyten.
Figur 5 demonstriert die Funktion der genetisch veränderten Monozyten im Körper.

Vorzugsweise ist die erfindungsgemäße antigen-präsentierende Zelle ein Monozyt, eine dendritische Zelle und/oder ein Makrophage.

Die APCs sind die Schaltstellen des anpassungsfähigen Immunsystems. Nur sie können eine solche Immunantwort auslösen. Dies sei in folgenden Abbildungen am Beispiel von Monozyten und der durch T-Helfer-Zellen ausgelösten Antikörperproduktion gezeigt:

Innerhalb des Immunsystems spielen die Antikörper normalerweise als Abwehrmoleküle ("humorale Immunreaktion") eine wichtige Rolle. Sie werden von ausgereiften B-Lymphozyten produziert. Die Induktion und Produktion der Antikörper erfolgt allerdings nicht unabhängig von den restlichen Zellen des Immunsystems; vielmehr wird diese humorale Immunreaktion von anderen Zellen des Immunsystems gesteuert.

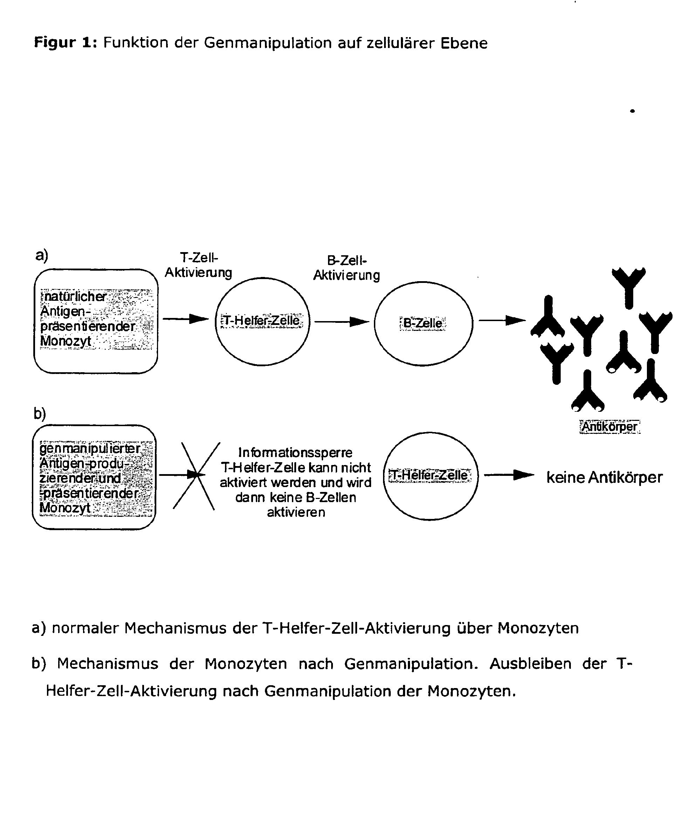

Die Antikörperproduktion - und so auch die Produktion der pathologischen Autoantikörper - lässt sich nicht aufrechterhalten ohne die Hilfe und Vermittlung von Monozyten und T-Helfer-Lymphozyten, die ihrerseits antigenspezifisch die B-Lymphozyten aktivieren (Fig.1).

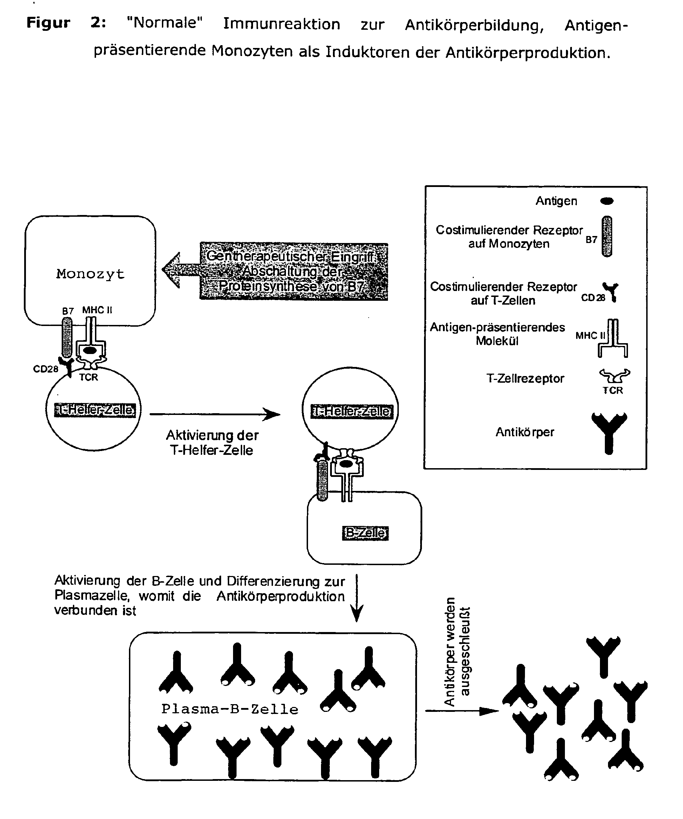

Die molekularen Mechanismen der Interaktion - des Zell-Zell-Kontakts - von Monozyten mit den T-Helfer-Lymphozyten ist auf Rezeptorebene bis ins Detail bekannt (Figur 2).

Handelt es sich bei dem Antigen z.B. um ein Bakterienprotein, ist die Immunreaktion für den Organismus nützlich. Ist das Antigen allerdings eine körpereigene Struktur, spricht man von einer (pathologischen) Autoimmunreaktion.

Neben dem zu präsentierenden Antigen (gegen welches sich die zu produzierenden Antikörper richten) sind auf der Zelloberfläche verschiedene Rezeptoren und Hilfsrezeptoren am Zell-Zell-Kontakt und der Zellaktivierung beteiligt. Ein essentielles Molekül der interzellulären Wechselwirkung bei der Antigenpräsentation (Kontakt von Monozyt mit den T-Helfer-Lymphozyten) ist ein costimulierender Rezeptor mit der Bezeichnung B7 (Figur 2)

Die erfindungsgemäße antigen-präsentierende Zelle weist vorzugsweise durch eine Transformation einer antigen-präsentierenden Zelle mit nukleinsäurehaltigem Material eine erhöhte Expression eines Antigens auf, wobei die antigen-präsentierende Zelle im wesentlichen nur eine Sorte eines Antigens präsentiert.

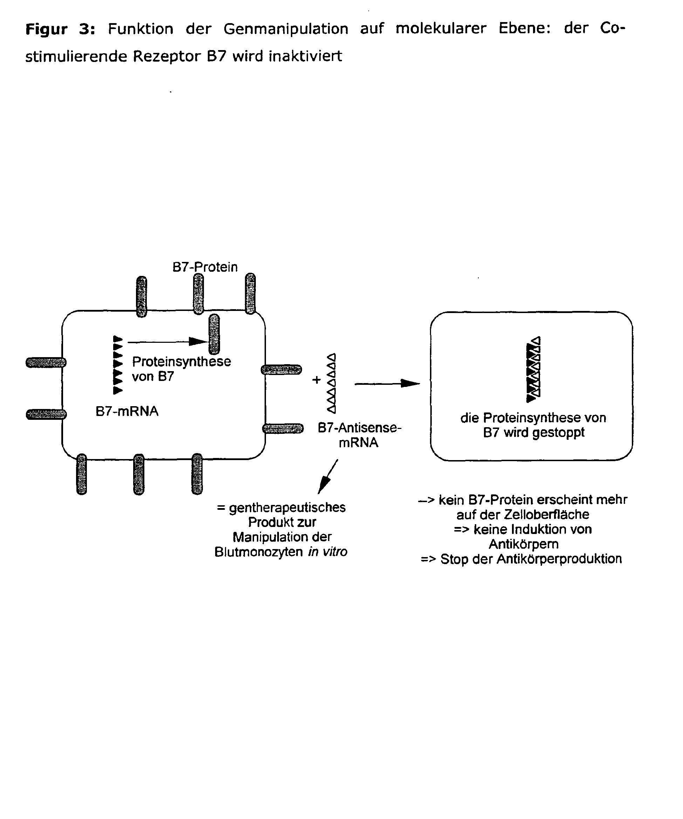

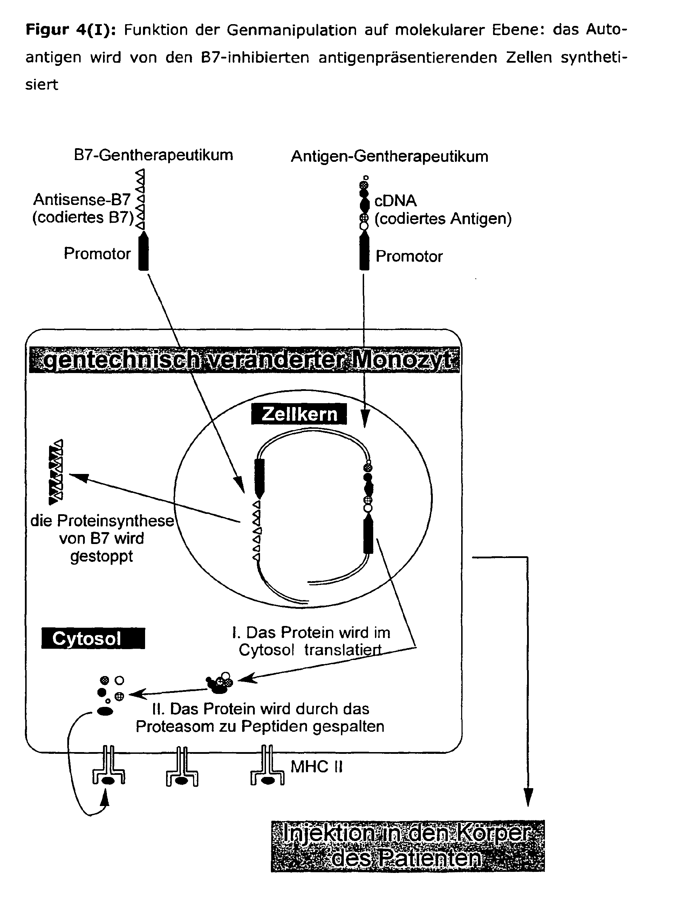

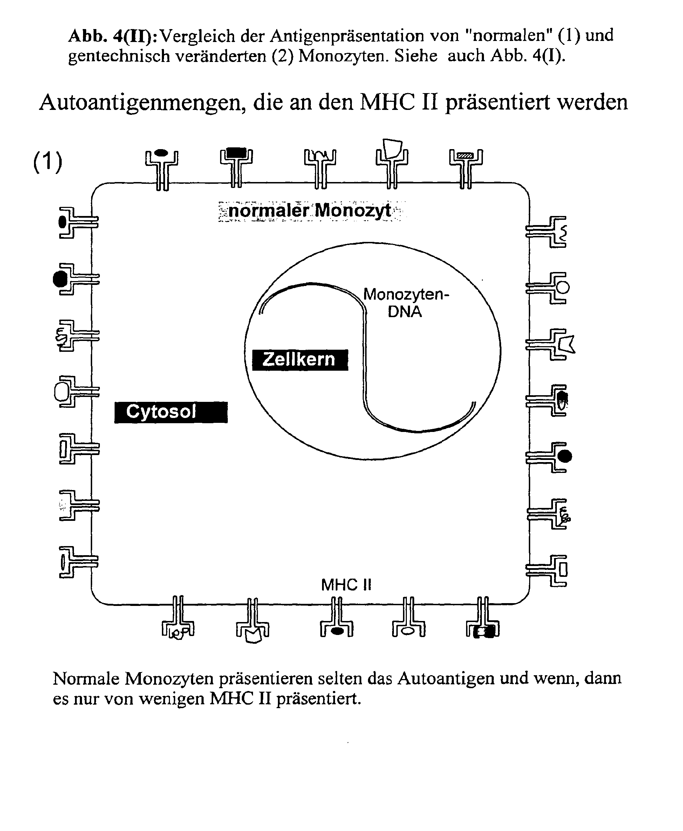

Das konzipierte gentherapeutische Verfahren beruht auf zwei parallelen gentechnologischen Eingriffen an patienteneigenen Blutmonozyten. Die Eingriffe erfolgen mittels geeigneter Sonden und bewirken die Verminderung von B7-Molekülen durch die Behinderung und damit der Verhinderung der Ausbildung des Moleküls auf der Oberfläche der Monozyten (Figur 3) und gleichzeitig eine starke Präsentation des Autoantigens (Figur 4).

### Verminderung der spezifischen Antikörperproduktion durch Gentherapie

Die Produktion pathologischer Autoantikörper wird durch die Manipulation von Monozyten spezifisch abgeschaltet. Der Co-Rezeptor B7, ohne den die Antigenpräsentation bzw. die Induktionskaskade zur Antikörperproduktion nicht anläuft, wird unterdrückt. Hinter dem Begriff B7 verbergen sich zwei unterschiedliche Co-Rezeptoren, die als CD80 (B7-1) und CD86 (B7-2) bezeichnet werden. Ihre Strukturen sind bekannt.

Nach dem Abschalten der Antikörperproduktion verschwinden die im Blut zirkulierenden Autoantikörper aufgrund des natürlichen Abbaus und des fehlenden Nachschubs.

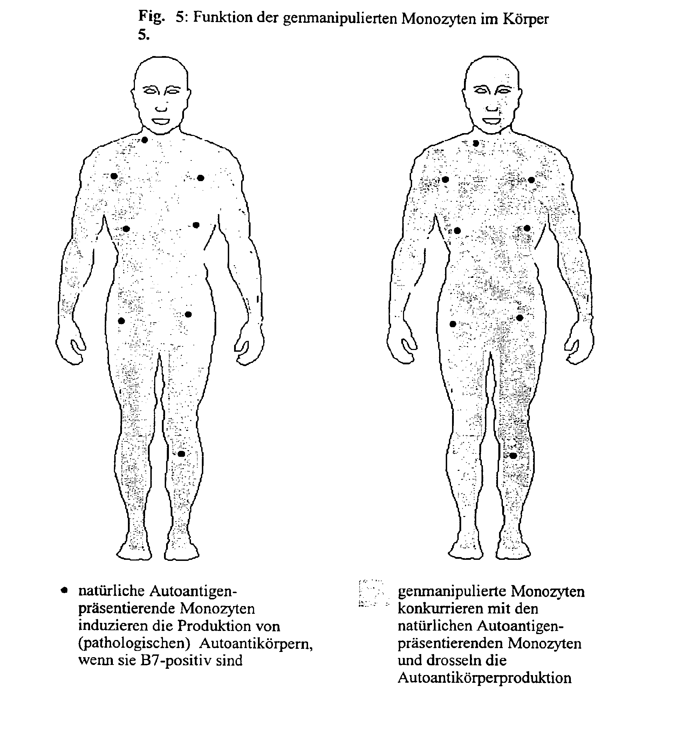

Nach der *in vitro* Manipulation der Monozyten werden die Zellen in die Blutbahn des Patienten zurückgegeben. Die genmanipulierten Monozyten schalten nunmehr die im Organismus befindlichen entsprechenden T-Helfer-Lymphozyten ab. Die genmanipulierten Zellen treten dabei unmittelbar in Konkurrenz zu den bereits im Organismus (vorzugsweise Blutbahn und Lymphsystem) vorhandenen Autoantigen-präsentierenden Monozyten, die allerdings B7 auf der Zelloberfläche tragen und normalerweise die T-Helfer-Lymphozyten und damit die Antikörperproduktion aktivieren (Figur 5).

### Antigenpräsentation bei gentechnisch veränderten Monozyten

Die cDNA eines Proteins, das als Autoantigen die Produktion pathologischer Autoantikörper hervoruft, wird in das Monozytengenom integriert. Diese Erbinformation dient dann zur Überproduktion des Autoantigens. Peptide dieses Autoantigens werden daraufhin bevorzugt an MHC II und/oder MHC I präsentiert (siehe auch Figur 4(II)). MHC II präsentiert die Peptide den T-Helferzellen und versucht solche zu finden, die spezifisch die präsentierten Proteine erkennen. Wenn der Co-Rezeptor B7 nicht gleichzeitig an der Zelloberfläche erscheint, werden die T-Helferzellen stillgestellt und unterliegen einem vorzeitigen Zelltod.

Alle genmanipulierten Monozyten präsentieren an den meisten ihrer MHC II-Komplexe das Autoantigen, während *in vivo* nur sehr wenige "normale" Monozyten das Autoantigen präsentieren und dann auch nur an wenigen MHC II-Komplexen. Es ist Ziel der Behandlung, *in vivo* die "normalen" Monozyten, die das Autoantigen präsentieren und die T-Helfer-Zellen aktivieren, zu verdrängen durch die auf Abschaltung der Antikörperprodukte programmierten, genmanipulierten Monozyten.

Die erfindungsgemäße antigen-präsentierende Zelle kann insbesondere eine erhöhte Anzahl von Homing-Rezeptoren, wie CD44, aufzeigen. Eine Überexpression von Homing-Rezeptoren wird der Antigen-präsentierenden Zelle den Weg in die Lymphknoten weisen, wodurch die genmanipulierten APCs sich vermehrt und schneller in Lymphknoten ansammeln. Die Lymphknoten sind der Ort, an dem die allermeisten Reaktionen des anpassungsfähigen Immunsystems hervorgerufen werden. Dort entfalten die genmanipulierten APCs daher eine um ein vielfaches höhere Wirkung als außerhalb der Lymphknoten.

In einer bevorzugten Ausführungsform der erfindungsgemäßen antigen-präsentierenden Zelle bewirkt eine Transfon-nation eine Erhöhung der Anzahl von Homing-Rezeptoren und/oder eine Suppression von Funktionen der B7-, CD40-Rezeptoren.

Insbesondere mit Antisense-Nukleinsäuren kann die B7- und/oder CD40-Rezeptor Expression in der erfindungsgemäßen antigen-präsentierenden Zelle verhindert oder reduziert werden.

Alternativ kann mit Nukleinsäuen eine Unterdrückung der Expression der B7-und/oder CD40-Rezeptoren durch Co-Suppression in der erfindungsgemäßen antigen-präsentierende Zelle bewirkt werden.

Die erfindungsgemäße antigen-präsentierende Zelle kann Nukleinsäuren enthalten oder transformiert sein, die eine Expression von mit B7-und/oder CD40-Rezeptoren affinen Strukturen aufweisenden Proteinen oder Peptiden bewirken. Damit werden Proteine gebildet, die durch Komplexbildung mit B7 - und/oder CD40-Rezeptoren praktisch neutralisieren. Insbesondere kommen dazu CTLA4, CD28, Antikörper, F(ab)₂, scFv und/oder F_{ab}-Fragmente als Proteine in Betracht.

In einer weiteren bevorzugten Ausführungsform enthält die erfindungsgemäße antigen-präsentierende Zelle Nukleinsäuren, die für eine Signalsequenz kodieren oder Expressionsprodukte einer Signalsequenz, die den Verbleib der Expressionsprodukte im endoplasmatischen Retikulum, dem Golgi-Apparat, dem Trans-Golgi-Netzwerk oder intrazellulären Vesikeln bewirkt.

Die erfindungsgemäße antigen-präsentierende Zelle wird zur Expression von Antigenen mit Nukleinsäuren transformiert, die den Transport der exprimierten Antigene in MHC II-Kompartimente der Zellen ermöglichen.

Alle genmanipulierten Monozyten präsentieren an den meisten ihrer MHC-Komplexe das Autoantigen, während nur sehr wenige "normale" Monozyten überhaupt das Autoantigen präsentieren und dann auch nur an wenigen MHC-Komplexen. Es ist Ziel der Behandlung, die "normalen" Monozyten, die das Autoantigen präsentieren und die T-Helfer-Zellen aktivieren, zu verdrängen durch die auf Abschaltung der Antikörperprodukte programmierten, genmanipulierten Monozyten. Es ist sehr wichtig, das Antigen (die Antigene) auch in einer Form zu verabreichen, die den Transport in die MHC II-Endosomen ermöglicht. Nur so kann zuverlässig eine Präsentation an MHC II erreicht werden. Bei einem gentechnischen Eingriff erfolgt dies in der Regel durch Manipulation des offenen Leserasters, so daß dem Leseraster eine Signalsequenz für dieses Kompartiment vorgeschaltet wird. Eine genetische Manipulation hat den zusätzlichen Vorteil, daß sie eine viel längere Halbwertzeit hat, als z.B. Oligonukleotide oder Peptide. Eine stabile Integration von Genen führt sogar zu einer permanenten Veränderung der Eigenschaft der Zielzellen.

Die entsprechenden Nukleinsäuren können dabei DNA, RNA, Oligonukleotide, Polynukleotide, Ribozyme, Peptidnukleinsäuren (PNA) sein.

Vorzugsweise besitzt die DNA Regulationselemente wie Enhancer, Promotoren, polyA-kodierende 3'-Enden zur Transkription der DNA in RNA, die RNA Regulationselemente zur Translation der RNA in Protein. Die Regulationselemente sorgen für eine effiziente Expression der Gene.

Die Herstellung der erfindungsgemäßen antigen-präsentierenden Zelle erfolgt zum Beispiel durch ex vivo Verfahren. Dabei wird vorzugsweise eine antigen-präsentierende Zelle ex vivo durch Behandlung mit Viren viralen Vektoren, bakteriellen Vektoren, Plasmiden, die durch Elektroporationstechniken, Iontophorese, ballistischen Methoden und/oder anderen Techniken zur Einschleusung von Molekülen in eine antigen-präsentierende Zelle transformiert.

In einer weiteren Ausführungsform kann eine antigen-präsentierende Zelle durch Behandlung mit Viren, viralen Vektoren, bakteriellen Vektoren, Plasmiden, die durch Elektroporationstechniken, Iontophorese, ballistischen Methoden und/oder anderen Techniken zur Einschleusung von Molekülen in eine Zelle mit supprimierter Funktion co-stimutatorischer Rezeptoren transformiert werden oder die Expression co-stimulatorischer Rezeptoren durch Verhinderung von deren Expression oder die co-stimulatorischen Rezeptoren werden durch Reaktion mit affinen Strukturen an einer Stimulation von T-Zellen, die an die antigen-präsentierende Zelle gebunden sind, gehindert.

Für die Unterdrückung der Co-Stimulation, die letztendlich ein Unterdrücken der Produktion eines oder mehrerer Proteine oder die Behinderung des Proteins oder der Proteine bedeutet, kommen verschiedene Strategien in Frage:
1. Antisense-Ansatz
2. Co-Suppression
3. Bindung des co-stimulatorischen Moleküls.

- Zu 1.:: In diesem Fall müssen Antisense-Nukleinsäuren in Kontakt mit der mRNA des co-stimulatorischen Moleküls kommen. Sie binden dann wahrscheinlich das Molekül und verhindern die Translation. Als Nukleinsäuren kommen eine große Bandbreite von Molekülen, wie RNAs, DNAs, PNAs, Ribozyme, in Frage. Auch hier würde ein gentechnischer Eingriff für die größte Halbwertszeit des Effekts sorgen.
- Zu 2.:: Es hat sich inzwischen herausgestellt, dass man die Produktion eines Genproduktes auch durch Integration einer möglichst homologen Sense-Gensequenz erreichen kann. Der Mechanismus ist noch völlig unbekannt.
- Zu 3.:: Die Bindung des co-stimulatorischen Moleküls z.B. durch spezifische Antikörper verhindert, daß dieses Molekül Kontakt mit dem avisierten Rezeptor auf einer T-Zelle aufnimmt und verhindert so die Aktivierung der T-Zelle. Die externe Zugabe solcher Bindemoleküle hat den Nachteil, dass sie auf alle Antigen-präsentierenden Zellen wirken und damit jede Immunreaktion verhindern. Um Immunreaktionen spezifisch zu behindern, haben wir ein Modell entworfen, bei dem die co-stimulatorischen Moleküle bereits in der Zelle, in einem intrazellulären Kompartiment gebünden werden. In einer Erweiterung dieses Modells soll dafür gesorgt werden, dass das Bindemolekül im intrazellulären Kompartiment zurückgehalten wird, so dass der co-stimulatorische Rezeptor erst gar nicht zur Plasmamembran gelangt. Hierfür sind Signalsequenzen bekannt, die dem offenen Leseraster des Bindemoleküls zugefügt werden müssen. Dieser intrazelluläre Ansatz lässt sich gut an isolierten Zellen durchführen. Auch hier ist ein gentechnischer Eingriff zu bevorzugen.

Die gewünschten Effekte können auch erreicht werden, wenn nur eines der beiden Ziele mit einem gentechnischen Eingriff vorgenommen wird. In diesem Fall können statt der Gene z.B. folgende anderen Moleküle eingesetzt werden:
1. Behinderung der Co-Stimulation durch
   Nukleinsäuren (zumeist komplementär zur Zielsequenz) bei denen es sich z.B. um Oligonukleotide, Polynukleotide, Ribozyme, Peptidnukleinsäuren (PNAs) handeln kann,
   Antikörper oder andere Moleküle, die die co-stimulatorischen Moleküle binden.
2. Antigen Kontaktierung durch
   Proteine, Peptide, Peptidomimetica.

Es werden nach dem erfindungsgemäßen Verfahren insbesondere Moleküle wie Antikörper, Proteine, Peptide, Peptidomimetica, CTLA4, CD28, CD40L und/oder Bestandteile und/oder Kombinationen dieser Moleküle, die z.B. B7-1, B7-2, CD40 binden, welche eine in Gegenwart einer Antigenpräsentation stattfindende Co-Stimulation der T-Zelle behindert, mit der antigen-präsentierenden Zelle in Kontakt gebracht.

Es kann vorteilhaft sein, die Moleküle mittels Vehikeln, wie Liposomen, Hydrogele, Zyklodextrine, biologisch abbaubare Nanokapsein, bio-adhäsive Mikrokugeln und/oder durch Elektroporationstechniken, Iontophorese, ballistische Methoden und/oder andere Techniken zur Einschleusung von Molekülen in die antigen-präsentierende Zelle Zelle zu transferieren.

Nukleinsäuren können insbesondere durch Viren, virale Vektoren, bakterielle Vektoren, Plasmide, die durch Elektroporationstechniken, Iontophorese, ballistische Methoden und/oder andere Techniken zur Einschleusung von Molekülen in die antigen-präsentierende Zelle transferiert werden.

Erfindungsgemäß beansprucht wird ein Arzneimittel, enthaltend mindestens eine erfindungsgemäße antigen-präsentierende Zelle. Vorzugsweise ist das erfindungsgemäße Arzneimittel als Infusionslösung zur intravenösen oder intraperitonealen Applikation formuliert. Die Formulierung ist so gewählt, dass bei Verabreichung des Arzneimittels keine wesentliche Beeinträchtigung der Wirksamkeit der erfindungsgemäßen antigen-präsentierenden Zelle erfolgt.

So kommt als Infusionslösung vorzugsweise physiologische Kochsalzlösung in Betracht. Grundsätzlich sind auch andere Lösungen, die einen pH-Wert von 5,5 bis 8,5 aufweisen, geeignet. Auch Serum, beispielsweise humanes Serum, authologes Serum oder Serum anderer Species, Lösungen mit Plasmaersatzstoffen, wie Polyvinylpyrolidon, kommen in Betracht. Typischerweise sollen 0,5 ml bis 500 ml appliziert werden. Diese Mengen sind selbstverständlich nicht absolut, sondern können vom Fachmann, je nach Bedingungen und Anforderungen, variiert und an die spezifischen Bedürfnisse eines Patienten angepasst werden.

Die erfindungsgemäße antigen-präsentierende Zelle kann insbesondere zur Herstellung eines Arzneimittels zur Behandlung von ungewollten Immunreaktionen, wie Autoimmunerkrankungen und Allergien oder gewollt hervorgerufenen Immunreaktionen, wie bei Immunisierungen verwendet werden.

Weiterhin kann die erfindungsgemäße antigen-präsentierende Zelle zur Herstellung eines Arzneimittels zur Behandlung von Immunreaktionen gegen allologe und/oder xenologe Gewebsmerkmale verwendet werden.

Erfindungsgemäß beansprucht werden insbesondere Verwendungen, bei denen die zu behandelnden Immunreaktionen in Verbindung mit Antigenen oder deren Gensequenzen und/oder Teilen davon stehen und ausgewählt sind aus der Gruppe bestehend aus
- Enzymen, deren Gensequenzen und/oder Teilsequenzen, insbesondere Glutamic acid decarboxylase (GAD), Rezeptor-Typ Protein Tyrosin Phosphatase IA-2Beta, Antigen: H⁺K⁺ATPase, U1RNP, Transglutaminase, Argininosuccinatlyase (ASL), Tyrosinase-related protein-2, Thyroid Peroxidase, Faktor VIII, Faktor IX;
- Rezeptoren, deren Gensequenzen und/oder Teilsequenzen, insbesondere Acetylcholinrezeptor vom Nicotintyp, β1-adrenerger-Rezeptor, α1-adrenerger-Rezeptor, Angiotensin-2-AT1-Rezeptor, Glutamat-Rezeptor, Thyrotropin-stimulierendes Hormon (TSH)-Rezeptor, LFA-1, HLA-B27, Epididymal Protein DE, Zona Pellucida (ZP)-3 Glycoprotein, Zona Pellucida (ZP)-4 Glycoprotein, Follicle-Stimulating Hormone (FSH) Rezeptor, Sperm Immunogen SP-10 oder Sperm Protein SP-10;
- Hormone oder Botenstoffe, deren Gensequenzen und/oder Teilsequenzen, insbesondere Insulin, Thyroglobulin, Follicle-Stimulating Hormone (FSH), Prostaglandin F2 alpha, Gonadotropin-Releasing Hormone (GnRH), Oestradiol-17beta, Oestrogen, Luteinizing Hormon (LH) Rezeptor, Inhibin, Testosteron, Androgen, Chorionic Gonadotrophin (CG), Interleukine, Interferone, Cytokine, Chemokine, Bone Morphogenetic Factors, β-Interferon, Estradiol;
- Strukturproteine, deren Gensequenzen und/oder Teilsequenzen, insbesondere Myelin Basic Protein (MBP), Proteolipid protein (PLP), Myelin oligodendrocyte glycoprotein (MOG), α-Fodrin, Nicht-erythroides α-Spectrin, Beta-Amyloid Precursor Protein (beta-APP), Typ 2 Kollagen, Sperm Plasma Membran Protein PH-20;
- Antigene, deren Gensequenzen und/oder Teilsequenzen, insbesondere CENP-A Autoantigen, Beta2GP-I, ribosomales P Protein, Ro/SSA, La/SSB, Sm/RNP, Sm, Scl-70, Jo-1, BCOADC-E2, Albumin, Glucagon, Inselzellantigene, Retinal S Ag;
- Allergene, die eine IgE-Antwort auslösen, deren Gensequenzen und/oder Teilsequenzen, insbesondere Der f 1, Der f 2, Der f 3, Der p 1, Der p 2, Der p 3, Der p 4, Der p 5, Der p 8, Eur m 1, Lep d 2, Fel d 1, Can f 1, Can f 2, Mus m 1, Rat n 1, Bla g 1, Bla g 2, Bla g 4, Bla g 5, Per a 1, Bienengift Phospholipase A₂ (PLA₂), Group V major allergen Phl p 5b von Timothy Grass Pollen, Hom s 1.

Weiterhin wird erfindungsgemäß beansprucht eine Verwendung bei der die zu behandelnden Immunreaktionen in Verbindung mit allologen und/oder xenologen Gewebsmerkmalen, deren Gensequenzen und/oder Teilsequenzen, insbesondere MHC I, MHC II, Rhesus Faktor stehen.

### Entwicklung einer Gentherapie

Das hier vorgestellte Verfahren zur Reduzierung von Immunantworten z.B. zur Behandlung von Autoimmunerkrankungen mit klar definiertem Autoantikörperprofil basiert auf der gentechnischen Manipulation von bestimmten Blutzellen vorzugsweise außerhalb des Körpers, die dann anschließend wieder in den Organismus zurückgeführt werden. Ziel dieser Behandlung ist unter anderem das spezifische Abschalten einer chronischen, durch das Immunsystem getriebenen Produktion von Autoantikörpern, welche die Krankheit auslösen.

### Anwendungsgebiete der Gentherapie

Das Verfahren zur spezifischen Abschaltung von Immunreaktionen ist immer dort sinnvoll, wo ein oder mehrere molekular definierte Targets (Antigene, Autoantigene) bekannt sind. Diese können beispielsweise mit einer Autoimmunerkrankung oder Allergie assoziiert seien.

Z.B. handelt es sich dabei um Krankheiten mit Autoantikörper-vermittelten Autoimmunreaktionen, d.h. um Krankheiten, bei denen die "Bindungsstrukturen" dieser Autoantikörper (Autoantigene, Targets) bekannt sind und pathogenetische Bedeutung haben.

Beispiele für Krankheiten mit bekannten, die Autoantikörper-bindenden molekularen Strukturen (Epitope) sind:
- Myasthenia gravis (Autoantikörper gegen den Acetylcholinrezeptor)
- Morbus Basedow (Autoantikörper gegen den TSH-Rezeptor der Schilddrüse)
- Dilatative Cardiomyopathie (DCM, Autoantikörper gegen den β1-adrenergen Rezeptor der Herzmuskelzellen)
- bestimmte Formen des insulinabhängigen Diabetes (Autoantikörper gegen Insulin)
- bestimmte Formen des malignen Bluthochdrucks (Autoantikörper gegen den Angiotensin- und/oder α1-adrenerger-Rezeptor.

### Behandlung mit genmanipulierten patienteneigenen Blutzellen: Prinzip der vorgeschlagenen Gentherapie

Innerhalb des Immunsystems spielen die Antikörper als Abwehrmoleküle ("humorale Immunreaktion") eine wichtige Rolle. Sie werden von ausgereiften B-Lymphozyten produziert. Die Induktion und Produktion der Antikörper erfolgt allerdings nicht losgelöst von den restlichen Zellen des Immunsystems; vielmehr wird diese humorale Immunreaktion von anderen Zellen des Immunsystems gesteuert. Die Immunreaktion lässt sich nicht aufrechterhalten ohne die Hilfe und Vermittlung von Antigen-präsentierenden Zellen und T-Helfer-Lymphozyten. Die molekularen Mechanismen der Interaktion - des Zell-Zell-Kontakts - von Antigen-präsentierenden Zellen mit den T-Helfer-Lymphozyten ist auf Rezeptorebene bis ins Detail bekannt.

Neben dem zu präsentierenden Antigen sind auf der Zelloberfläche verschiedene Rezeptoren und Hilfsrezeptoren am Zell-Zell-Kontakt und der Zellaktivierung beteiligt. Ein essentielles Molekül der interzellutären Wechselwirkung bei der Antigenpräsentation (Kontakt von Antigen-präsentierenden Zellen mit den T-Helfer-Lymphozyten) sind costimulierende Rezeptoren mit den Bezeichnungen B7-1 und B7-2. Auch CD40 spielt bei dieser Signaltransduktion eine Rolle.

Die Funktionen von B7 und CD40 sind Inhalt zahlreicher Publikationen (Daikh *et al*. 1997; Greenfield *et al.* 1998; Johnson-Leger *et al.* 1998; McAdam *et al.* 1998; Vyth-Dreese *et al.* 1995) und werden auch umfangreich in Lehrbüchern abgehandelt (s. z.B. Janeway and Travers 1997).

Das konzipierte Verfahren beruht auf zwei parallelen Eingriffen an patienteneigenen Antigen-präsentierenden Zellen. Die Eingriffe erfolgen mittels geeigneter Sonden und bewirken
- die Abschaltung von B7 und damit die Verhinderung der Ausbildung des Moleküls auf der Oberfläche der Antigen-präsentierenden Zellen und/oder die Abschaltung von CD40 und
- gleichzeitig eine starke Präsentation des Autoantigens auf den Antigen-präsentierenden Zellen.

Sollte die Manipulation der Zellen, die z.B. Monozyten sind, *in vitro* erfolgen, werden die Zellen in die Blutbahn des Spenders zurückgegeben. Die genmanipulierten Monozyten schalten nunmehr die im Organismus befindlichen entsprechenden T-Helfer-Lymphozyten ab. Die genmanipulierten Zellen treten dabei unmittelbar in Konkurrenz zu den bereits im Organismus (vorzugsweise Blutbahn und Lymphsystem) vorhandenen Autoantigen-präsentierenden Monozyten, die allerdings B7 auf der Zelloberfläche tragen und normalerweise die T-Helfer-Lymphozyten und damit unter anderem die Antikörperproduktion aktivieren.

### Behandlung von Autoimmunerkrankungen, Allergien und Transplantationen: Stand der Technik

Kausaltherapien zur Behandlung von Autoimmunerkrankungen und Allergien existieren nicht. Von wenigen Ausnahmen abgesehen (extrakorporale Elimination der Antikörper aus dem Blut der Patienten bzw. Plasmapherese), erfolgt die Behandlung von Autoimmunerkrankungen und Allergien medikamentös durch eine Hemmung von Reaktionen des Immunsystems.

Nach wie vor am gebräuchlichsten ist die medikamentöse Behandlung von Autoimmunerkrankungen, Allergien und Transplantationen mit immunsuppressiven Präparaten wie Cortison und dessen Abkömmlingen, Cyclosporin, Beta-Interferon oder Zytostatika (Methothrexat). Desweiteren werden Antikörper, Antagonisten und Oligonukleotide gegen verschiedene Signalkomponenten des Immunsystems mit dem Zweck erprobt, die Aktivierung von T-Zellen zu verhindern. Solche Arzneimittel sind bestenfalls selektiv, nie aber spezifisch gegen die falsch programmierten Autoimmunreaktionen gerichtet. Immunsuppressiva haben daher auf wichtige, notwendige und nützliche Teile des Immunsystems eine negative Wirkung; aus diesem Grund und ihrer Nebenwirkungen wegen ist ihr Einsatz begrenzt.

Von den immunsuppressiven Therapien unterscheidet sich das vorgelegte Konzept einer Gentherapie zur Reduktion von spezifischen Immunreaktionen grundlegend. Es wird eine spezifische Abschaltung fehlgeleiteter immunologischer Reaktionen durch gentechnisch umprogrammierte patienteneigene Blutzellen durchgeführt.

Die aufgezeigte Methodik ist als allgemein gültiges Konzept zur Reduzierung von Immunantworten gedacht. Es sollte mit diesem Grundkonzept möglich sein jede Immunreaktion des anpassungsfähigen Immunsystems wieder abzustellen. Außerdem können nach belieben solche Immunreaktionen hervorgerufen und dann wieder abgestellt werden.

### Somatische Gentherapie

### Grundlagen

Als Hilfsmittel für den Gentransfer werden hauptsächlich retrovirale (Hunt 1987; Porter 1996) oder adenovirus-assoziierte Vektoren eingesetzt (Bertran 1996), liposomen-vermittelter Gentransfer spielt nur eine untergeordnete Rolle.

### Einteilung der Retroviren

Retroviren sind einzelsträngige, umhüllte RNA Viren. Die Familie der Retroviridiae wird in drei Unterfamilien eingeteilt: Onkoviren, Lentiviren und Spumaviren (Fields 1996). Zu den Lentiviren gehören HIV-1 und HIV-2, zu den Spumaviren das Simian Foamy Virus. Die Gruppe der Onkoviren wird in 5 Genera unterteilt (Tabelle 1).

**Tab. 1: Einteilung der Onkoviren und ihre wichtigsten Vertreter bei Menschen und Tieren**

| Genus | Mensch | Tier |
|---|---|---|
| B-Typ Viren | HervK Familie | Maus Mamma Tumor Virus (MMTV) |
| C-Typ Viren (Säuger) | Erv-3 S71 Familie | Moloney Maus Leukämie Virus(MoMuLV) |
| | | Harvey Maus Sarkom Virus (HaMSV) |
| | | felines Leukämie Virus (FeLV) |
| | | Gibbon Ape Leukämie Virus (GALV) |
| | | Affen Sarkom Virus (SSV) |
| ALV-related | | aviäres Leukose Virus (ALV) |
| (C-Typ Viren) | | Rous Sarkom Virus (RSV) |
| | | Rous assoziertes Virus (RAV 1-50) |
| D-Typ Viren | | Mason Pfitzer Affen Virus |
| HTLV/BLV Gruppe | humane T- Zell Leukämie Viren (HTLV1/HTLV2) | Rinder Leukämie Virus (BLV) |

### Genereller Aufbau der Retroviren

Elektronenmikroskopische Bilder zeigen, daß Retroviren umhüllte Partikel mit einer Grösse von etwa 100 nm sind. Das Capsid ist von einer Membran umgeben, in die nach außen weisende Hüllproteine (env) eingelagert sind. Innen ist die Membran mit einer Schicht von Matrixproteinen (MA) versehen. Im Innern des Partikels befindet sich das Nucleocapsid, welches aus Capsidproteinen (CA) aufgebaut ist. Matrixprotein und Capsidprotein gehören zu den sogenannten gruppenspezifischen Antigenen (gag). Innerhalb des Capsids befinden sich zwei Moleküle einzelsträngige RNA sowie die Enzyme Reverse Transkriptase (RT), Integrase (IN) (durch die als pol bezeichneten Sequenzen kodiert) und
Protease (PR) (durch die als pro bezeichneten Sequenzen kodiert) (Fields 1996).

Das Genom der Retroviren besteht aus zwei identischen Einzetstrang-RNAs von 7 - 10 kb Länge.

Die RNA beginnt mit der m⁷GPPP Cap Struktur, die wichtig für die Translation der Strukturproteine ist, und endet mit der ca. 200 Basen langen Poly(A) Sequenz. Zwischen Cap Struktur und Poly(A) Sequenz findet man regulatorische Sequenzen, unter anderem den Spleißdonor, dann folgen die für die gag-, pro- und pol-Proteine kodierenden Sequenzen, der Spleißakzeptor und die kodierende Sequenz für das env Gen.
Die einfachen Retroviren, wie z.B. die C-Typ Viren, benötigen nur diese Proteine für ihren Lebenszyklus. Komplexere Viren,wie z.B. die Lentiviren, enthalten noch weitere Gene, entweder durch Verschiebung der Leserahmen oder durch weitere Sequenzen zwischen der env-Region und den 3' regulatorischen Sequenzen.

### Maus Leukämie Viren

Das für den Gentransfer am häufigsten benutzte Virus, das amphotrope Maus Leukämie Virus (MuLV), gehört zur Klasse der C-Typ Viren. Murine Leukämie Viren werden anhand ihrer Rezeptornutzung und damit ihres Wirtsbereiches weiter unterteilt (Tab. 2).

**Tab.2: Einteilung der murinen Leukämie Viren**

| Klasse | Wirtsbereich | Rezeptor | Funktion |
|---|---|---|---|
| amphotrop | alle Mammalia | Pit-2 (ram-1) | PO₄³⁻ Transporter |
| ecotrop | Maus | mCat | AS Transporter |
| polytrop | alle Mammalia | ? | ? |
| xenotrop | alle Mammalia au- ßer Maus | ? | ? |
| MCF | Maus | ? | ? |
| 10A1 | alle Mammalia | Pit-1 (Glvr-1) und Pit-2 (ram-1) | |

Zellen, die mit den Viren einer Gruppe infiziert sind, können nicht von Viren derselben Klasse überinfiziert werden, da die zellulären Rezeptoren bereits von den env Proteinen dieses Virus besetzt sind. Dieses Phänomen wird Interferenz genannt. Die Zellen können jedoch mit Viren einer anderen Klasse infiziert werden.

### Aufbau des MuLV -Genoms

Anschließend an die 5' Capping Region befindet sich die 5' R Region, gefolgt von der U5 Region, der Primer Binding Site (PBS), dem Leader, den Sequenzen für die viralen Proteine, dem Polypurintrakt, der U3 Region, der 3' R Region und der Poly A Sequenz.
Direkt an die Capping Struktur anschließend und vor dem Poly A Strang befinden sich jeweils die beiden identischen R Regionen. Sie sind ca. 60 Basenpaare lang und wichtig für die reverse Transkription.

Die am 5' Ende anschließende U5 Region ist die erste transkribierte Region bei der reversen Transkription und enthält wichtige Sequenzen für die Initiation der Transkription. Sie ist ca. 75 Basen lang und wird zum 3' Ende der 5' Long Terminal Repeats (LTR) des Provirus.
Die Primerbinding Site ist 18 Nucleotide lang. Hier wird ein Molekül tRNA als Primer gebunden.
Die Leader Sequenz ist ca. 475 Basen lang und enthält die Splice Donor Sequenzen. Außerdem ist das Verpackungssignal hier lokalisiert. Es bewirkt beim Zusammenbau der Virionen die Verpackung der genomischen RNA in die Virionen, während andere RNA nicht verpackt wird.
Daran schließen sich die Sequenzen für die gag, pro und pol Gene an, gefolgt vom Splice Akzeptor und den Sequenzen für die env Gene.
An die env Sequenz schließt sich direkt vor der U3 Region der Polypurin Bereich an. Hier ist die Initiationsstelle für die Plus Strang Synthese lokalisiert.
Die U3 Region ist ca. 500 Basen lang und wird der 5' Bereich der LTR im Provirus. Sie enthält alle für die Transkription der viralen Proteine erforderlichen Sequenzen, wie z. B. Bindungsstellen für verschiedene Transkriptionsfaktoren oder die TATA Box. Zellinien spezifische Regulation der Expression z. B. wird von der U3 Region codiert.
Die U3 Region wird von der 3' R Region gefolgt, sowie dem Polyadenylierungs Signal und der 200 Nucleotid langen Poly A Sequenz.

### Aufbau der MuLVs

Die Matrix-, Capsid- und Nucleoproteine werden von zwei gemeinsamen Polyprotein-Vorläufern synthetisiert. Das Pr180^{gag-pol} enthäl neben den gag Proteinen auch die pol Domäne, das Pr65^{gag} enthält nur die gag Proteine. Diese Vorläuferproteine werden von der viralen Protease (PR) sequentiell in die reifen Proteine gespalten: Matrixprotein (p15), Capsidprotein (p30) und Nucleoprotein (p10); Protease (p14), Reverse Transcriptase (p80) und Integrase (p46).
Das Matrixprotein ist über aminoterminal angefügte Myristinsäurereste mit der Lipidmembran am engsten assoziert. Das Capsid oder Core ist aus dem hydrophoben Capsidprotein aufgebaut. Das kleine basische Nucleoprotein liegt innerhalb des Cores. Werden diese Proteine in eukaryontischen Zellen exprimiert, lagern sie sich dort in die Zytoplasmamembran und generieren nach Ausknospung fast normal aussehende Virionen. Die gag Proteine liegen im ersten Leserahmen des Virusgenom und werden in der Zelle in großen Mengen synthetisiert, da sie sowohl vom Pr180^{gag-pol} als auch vom Pr65^{gag} generiert werden (Fields 1996).
Die folgenden pro und pol Gene, die für die enzymatischen Aktivitäten verantwortlich sind, liegen im nächsten Leserahmen und werden in geringeren Mengen hergestellt (nur vom Pr180^{gag-pol}).
Die Protease ist verantwortlich für die Aufspaltung der gag und pol Polyprotein-Vorläufer in die reifen Proteine.
Die reverse Transkriptase enthält die DNA Polymerase-Aktivität und eine RNase H-Aktivität, sie besitzt jedoch keine "proof reading" Aktivität. Reverse Transkriptase gehört zu den am stärksten konservierten Bereichen des Retrovirusgenoms. Man findet nicht nur Übereinstimmung innerhalb der Retrovirus Familie, sondern bestimmte Bereiche sind auch bei genetisch weit entfernten Viren, wie z.B. den Hepadnaviren (doppelsträngige DNA-Viren) und dem Blumenkohl-Mosaic Virus zu finden.
Die Integrase ist das Enzym, das die provirale DNA in das Genom der Wirtszelle einfügt.
Die env Gene haben einen eigenen Leserahmen und werden über einen Spleißakzeptor transkribiert. Sie werden ebenfalls als Polyprotein-Vorläufer transkribiert (Pr80^{env}), aber durch eine zelluläre Protease gespalten: Transmembran Region (p15E) und externes Glykoprotein (gp70).
Das env Protein besteht aus zwei Polypeptidketten, die durch Disulfid -Brücken miteinander verbunden sind. Diese Ketten werden als gemeinsamer Vorläufer synthetisiert; der aminoterminale Teil ergibt die externe Polypeptidkette (SU), der carboxyterminale Teil die Transmembranregion (TM). Das SU Protein ist glykosyliert und für die Interaktion mit dem Rezeptor zuständig; das TM Protein ist nicht glykosyliert und sorgt für die Verankerung der Proteine in der Membran. Wahrscheinlich ist er auch für die Dimerisierung der env Moleküle verantwortlich.

Da die Bindung zwischen dem SU und dem TM Protein nicht besonders fest ist, geht das SU Protein häufig "verloren". Dies bedingt unter anderem die mangelnde Stabilität infektiöser Viren während der Ultrazentrifugation und die große Anzahl an infektionsdefekten Partikeln.

### Der retrovirale Lebenszyklus der MuLVs

Der Replikationszyklus des murinen Leukämie Virus entspricht dem allgemeinen Schema der Retroviren und unterteilt sich in folgende Schritte:
- Anheftung an den Rezeptor und Zelleintritt
- Reverse Transkription
- Kerntransport und Integration
- Synthese viraler RNA und Proteine
- Ausknospung und Reifung der Virionen

### Anheftung an den Rezeptor und Zelleintritt

Die Infektion einer Zelle erfolgt durch die spezifische Anlagerung des Virus an seinen Rezeptor auf der Zelloberfläche und die anschließende Aufnahme des Virus in die Zelle. Nur Zellen, die diesen Rezeptor exprimieren, können infiziert werden.
Die Interaktion zwischen dem zellulären Rezeptor und dem externen viralen Hüllprotein (SU) bewirkt eine Konformationsänderung bei letzterem und ermöglicht die Fusion mit der Zellmembran durch die Transmembrandomäne des Virus. Die Aufnahme der Virionen erfolgt entweder durch pH abhängige Fusion (ecotropes Virus) oder durch Endozytose (amphotropes Virus).

### Reverse Transkription

Im Cytoplasma der Zelle erfolgt das Uncoating der Viren; dadurch wird die virale RNA freigesetzt und die Reverse Transkriptase aktiviert. Das Capsid verbleibt jedoch in enger Assoziation mit der viralen RNA und ist notwendig für die korrekte Transkription der viralen RNA in DNA.
Ausgehend von dem tRNA Primer, der nahe dem 5'Ende an der Primerbindungsstelle (PB) sitzt, wird zunächst der Minus-DNA-Strang synthetisiert. Es wird die 5' gelegene U5 und R-Region transkribiert (strong stop DNA), wobei gleichzeitig durch die RNAse H Aktivität der Reversen Transcriptase die RNA Matritze abgebaut wird. Die so freigewordene DNA "springt" an das 3' Ende der viralen RNA und es erfolgt eine Anlagerung der neu synthetisierten R-Region an die dort vorhandene R-Region. Ausgehend von dieser Matritze wird die restliche virale RNA in DNA transkribiert und bis auf den RNAse H resistenten Polypurin Bereich vor der U3 Region abgebaut. Dieser Bereich dient am bereits synthetisierten Minus-DNA-Strang als Matritze für die Synthese des Plus-DNA-Stranges, dessen 3' Ende als erstes transkribiert wird. Dann werden die verbleibenden RNA Reste abgebaut und die Primerbindungsstelle des Plus-Stranges kann an die Primerbindungsstelle des Minus-Stranges binden und die Synthese des Plus-Stranges kann vollständig ablaufen. Als letzter Schritt werden beide DNA Stränge vervollständigt. Die neu synthetisierte DNA enthält an beiden Seiten die Long Terminal Repeats (LTR).

### Kerntransport und Integration

Die im Cytoplasma neu synthetisierte virale DNA wandert als Präintegrationskomplex in den Kern und lagert sich dort an die zelluläre DNA an. MuLV können nur sich teilende Zellen infizieren, da diese eine aufgelöste Zellmembran haben. Sie besitzen kein Kerntransportsignal oder Protein (wie z.B. HIV), daß den Kerntransport bewirken könnte. Der Präintegrationskomplex ist auch zu groß, um durch Kernporen zu diffundieren. Die virale DNA liegt in linearer Form vor. Die viruseigene Integrase spaltet die zelluläre DNA an einer beliebigen Stelle und erzeugt überhängende Enden von 6 Basenpaaren. Die 3' Enden der viralen DNA werden mit einem Strang der zellulären DNA ligiert; vorher werden jeweils 2 Basen an den 3' Enden der viralen DNA entfernt. Die fehlenden Sequenzen im zweiten Strang werden durch das zelleigene Reparatursystem aufgefüllt, wobei auch die beiden überzähligen Basen am 5' Ende der viralen DNA entfernt werden.

### Retrovirale Vektoren

Retroviren gehören zu den nicht lytischen Viren, haben ein einfaches Genom, sind gut charakterisiert, integrieren stabil in das Genom der infizierten Zelle und können dort dauerhaft das inserierte Gen exprimieren- alles Gründe, um sie für gentherapeutische Ansätze attraktiv zu machen.
Für die Transduktion von humanen Zellen werden Vektoren mit amphotropem env eingesetzt oder Pseudotypvektoren mit dem env anderer Viren. Es wird an der Entwicklung zelltypspezifischer Vektoren gearbeitet, entweder durch Wahl einer geeigneten LTR (Hunt 1987), eines besonderen Hüllproteins oder durch Einbringen von Antikörpern oder Rezeptoren in die Virushülle.

### Aufbau

Retrovirale Vektoren werden auf der Basis von Retroviren hergestellt. Um die Bildung von replikationskompetenten Viren zu verhindern, werden die viralen Gene deletiert. Anstelle dieser wird zwischen die LTRs das zu exprimierende Gen mit den nötigen Regulationssequenzen kloniert und mit dem Verpackungssignal versehen. Die Verpackung der Vektoren erfolgt in speziellen Verpackungszellinien, die alle zur Verpackung nötigen Proteine bereitstellen. Die zu transferierende DNA wird durch die Verpackungskapazität der Virionen begrenzt. Retrovirale Vektoren können bis 9 kb Fremd DNA übertragen.
Durch Wahl eines geeigneten LTR oder eines zusätzlichen Promotors wird versucht, eine möglichst hohe Expression des transferierten Gens in der gewünschten Zielzelle zu erhalten. Durch verschiedene Strategien können mehrere Gene von einem Vektor aus kodiert werden.

### Verpackungszellen für retrovirale Vektoren

Da bei retroviralen Vektoren alle viralen Gene deletiert wurden, müssen die für die Replikation benötigten Proteine von geeigneten Verpackungszellen bereit gestellt werden.
Die zur Zeit benutzten Verpackungszellen enthalten ein Konstrukt, das für die gag pol Gene kodiert, und ein weiteres, das für die Hüllproteine kodiert (Danos 1988). Sie sind stabil in das Genom der Zellen integriert. Die Konstrukte enthalten kein Verpackungssignal, so daß nur leere Virionen gebildet werden. Erst wenn Vektor DNA in den Zellen vorliegt, entstehen RNA haltige Viren.
Die Benutzung von zwei verschiedenen Konstrukten zur Expression von gag, pol und env soll die Gefahr der Bildung von replikationskompetenten Viren vermindern. Es sind mindestens drei Rekombinationsereignisse erforderlich, bevor ein replikationskompetentes Virus (RCR) entstehen kann. Trotzdem müssen Verpackungszellen für klinische Anwendungen ständig auf RCRs überprüft werden.

### Ausführungsbeispiel

### Methoden

Die grundsätzlichen Methoden der Molekularbiologie sind in "Current Protocols" (Fred M. Ausubel 1999; John E. Coligan 1999; Juan S. Bonifacino 1999) offenbart. Hierauf wird ausdrücklich Bezug genommen.

### Zellkultur

Alle Zellinien wurden in Brutschränken bei 37°C und 5 % CO₂ kultiviert.

### Isolation von Monozyten

Folgende Methoden wurden verwandt, um humane Blutmonozyten oder solche aus Mäusen aufzureinigen. Dem Fachmann sind noch weitere Methoden an sich bekannt.

### Isolierung und Kultur peripherer Blutmonozyten

- Medium 1:: Dulbecco's PBS ohne (Ca und Mg)
- Medium 2:: RPMI 1640
10% fetales Kälberserum
- Medium 3:: RPMI 1640, serumfrei

Periphere Blutmonozyten wurden aus *buffy coats* oder Mäuseblut vorzugsweise durch Dichtezentrifugation über ein Lymphozytentrennmedium und anschließende selektive Adhäsion isoliert.
*Buffy coats*, präpariert aus ca. 400 ml Zitratblut von gesunden Spendern, wurden von einer lokalen Blutspende erhalten. Frisch präparierte *buffy coats* wurden jeweils mit Medium 1 auf ca. 120 ml aufgefüllt. 30 bis 35 ml der Zellsuspension wurden in sterilen 50ml-Polypropylenröhrchen mit 15 ml Ficoll-Paque (d = 1,077 mg/ml) vorsichtig mit einer Spritze unterschichtet und bei 400 x g, 30 min, 24°C zentrifugiert. Die in der Interphase enthaltenen Lymphozyten/Monozyten wurden zweimal bei 200 x g, 15 min, 20°C gewaschen. Dann wurden die Zellen mit einer Dichte von 5x 10⁶/ml bis 2 x 10⁷/ml in sterile, gelatinebeschichtete Zellkulturschalen in Medium 2 ausgesät. Nach zweistündiger Inkubation bei 37°C konnten nicht adhärente Zellen durch dreimaliges, gleichmäßiges Waschen mit Medium 3 entfernt werden. Die Monozyten wurden dann in Medium 2 bei 37°C, 5% CO₂ kultiviert. Das Mäuseblut wurde in gleicher Weise behandelt.

Der Buffy coat bzw. das Mäuseblut wurde 1:4 mit PBS ohne (Ca und Mg) verdünnt, 15 Minuten bei 100 g zentrifugiert. Der obere gelbliche Teil (Plasma) enthält hauptsächlich Thrombozyten. Er wird vorsichtig abgenommen und verworfen. Der Rest wird mit PBS ohne Ca & Mg auf ca. 120 ml verdünnt. die Lösung wird auf vier Falcontubes mit jeweils 15 ml Ficoll (d = 1,077 mg/ml) überschichtet. Dann wird 20 Minuten bei 700 g ohne Bremse zentrifugiert. Die Interphase (milchiger Ring) wird abgesaugt (Lymphozyten, Monozyten, Thrombozyten) und zweimal gewaschen. Das Pellet (Erythrozyten, Granulozyten) wird verworfen.
Die Monozyten werden dann durch Adherenz an gelatinierte Plastikgefäße isoliert, da die anderen Zelltypen beim waschen entfernt werden. Hierzu wurde die Interphase zwei Stunden in den gelatinierten Plastikgefäßen inkubiert.

### Virusproduktion

Für transiente Virusproduktion wurden die Zellen unter Verwendung der Ca-PO₄-Methode transfiziert und die Überstände an drei aufeinanderfolgenden Tagen abgesammelt.
Bei stabilen Producer Linien wurden die Zellen konfluent ausgesät und das Medium nach 6 h gewechselt. Nach 16 h wurden die Überstände abgesammelt und durch einen 0,8 µm Filter filtriert. Der Virusüberstand von amphotropoen Verpackungslinien wurde direkt eingesetzt.

### Virusanreicherung

Zur Virusanreicherung wurden die Überstände der virusproduzierenden Zellen abgesammelt und durch einen 0,8 µm Filter filtriert. Anschließend wurden sie in ein Zentrifugationsröhrchen überführt und bei 100.000 g und 5°C 2 h abzentrifugiert. Der Überstand wurde entfernt und das pelletierte Virus in wenig DMEM-Medium mit 2 % BSA aufgenommen. Die konzentrierten Viren wurden entweder direkt in Teste eingesetzt oder bei -80°C eingefroren.
Zur Optimierung wurde in Zentrifugationsröhrchen 5 ml einer 15% Sucroselösung vorgelegt und diese mit dem Zellkulturüberstand überschichtet. Dann wurde wie oben beschrieben zentrifugiert.
Für Westernblot-Analysen wurde der Überstand auf zwei Röhrchen aufgeteilt und zentrifugiert. Eines der Virus-Pellets wurde in Medium aufgenommen zur Titerbestimmung, das andere wurde in der gleichen Menge Virus-Lysepuffer aufgenommen und bis zur Verwendung bei -80°C eingefroren.

### Virustiterbestimmung

### a.) GTU

Je 5x10³ SC-1 Fibroblasten wurden in 1 ml Medium in die Weil einer 24 Weil Platte ausplattiert. Nach 4 Stunden Inkubation wurde das Medium entfernt, durch 1 ml einer Virusverdünnung (1:5 Verdünnungsschritte, dreifach Ansatz) ersetzt und weitere 16 h inkubiert. Die Expression der zusätzlichen genetischen Informationen wurde im FACS oder per RT-PCR quantifiziert. Es wurden die Klone der Verdünnungsstufe ausgezählt, die 1 - 6 Klone pro Well enthielt.

### b.)Verdünnung der infizierten Zellen

Je 1x10⁶ SC-1 Fibroblasten wurden in 3 ml in die Wells einer 6-Well Platte ausplattiert. Nach 4 Stunden Inkubation wurde das Medium entfernt, durch 2 ml einer Virusverdünnung ersetzt und weitere 16 h inkubiert. Die Zellen wurden trypsiniert und in Verdünnungen von 10², 3x10² bis 1x10⁵ Zellen/Well in 4 ml Medium ausplattiert. Es wurden je zwei Well pro Verdünnung angesetzt. Die Expression der zusätzlichen genetischen Informationen wurde im FACS oder per RT-PCR quantifiziert. Es wurden die Klone der Verdünnungsstufe ausgezählt, die 1 - 6 Klone pro Well enthielt.
Die Klonierungseffizienz berechnete sich aus den Klonen der Klonierungskontrolle:
Der Virustiter berechnete sich aus den Klonen der einzelnen Virusverdünnung, der Verdünnungsstufe und der Klonierungseffizienz.
Zur Titerbestimmung wurden nur Verdünnungen verwendet, in denen die Anzahl der Klone linear mit den Verdünnungen anstieg.

### Verwendete therapeutische Gene

Als Genfähre diente ein amphotropes, murines Leukämievirus. Die in den Versuchen verwendeten Gene bezwecken einerseits die Reduktion der an der Zelloberfläche erscheinenden B7- und/oder CD40-Moleküle und sollen zusätzlich für die Expression eines Antigens sorgen. Dazu werden zu Beginn CTLA4Ig zur Unterdrückung von B7 und/oder CD40-Antisense-cDNA und ein Antigen verwendet. Bei dem Antigen handelt es sich bei humanen Monozyten um das C-Fragment des Tetanustoxins. Bei den Mäusemonozyten um Ovalbumin. CTLA4Ig ist ein Fusionsprotein aus dem Fc-Teil eines Antikörpers und dem Teil von CTLA4, der an B7 bindet. Es ist gezeigt worden, daß dieses Molekül sehr effektiv beide B7 (1 und 2)- Moleküle bindet und auch neutralisieren kann (Linsley, et al.; Patent; 1993).
Da Menschen gegen Tetanus geimpft werden, kann in humanem Blut eine starke Reaktion gegen Fragmente des Tetanustoxins (z.B. das C-Fragment) auch *ex vivo* beobachtet werden. Dies wurde genutzt, um die *ex vivo* T-Zellproliferation zu beobachten.
Die Mäuse wiederum können gegen Ovalbumin geimpft werden. Danach kann die Reduktion der Immunantwort gegen Ovalbumin beobachtet werden.
Als Promotor wurde der CMV-Promotor (Boshart *et al.* 1985; Nelson *et al.* 1987) verwendet. Die Gene waren durch IRES-Sequenzen transkriptional gekoppelt (Hildinger *et al.* 1999).

### Transfektion der Monozyten

Hierfür wurde der Überstand der Verpackungszellinie verwendet. Mit diesem wurden die Monozyten inkubiert.

### Ergebnisse mit humanen Monozyten, in vitro

Die Buffy Coats wurden vorweg auf ihre Reaktivität gegen Tetanustoxin getestet. Der Versuchsansatz enthält Tests, mit Monozyten und T-Lymphozyten. Die Monozyten wurden
1. nicht genetisch verändert;
2. mit einem Retrovirus behandelt, der keine therapeutischen Gene enthält;
3. mit einem Retrovirus behandelt, der mit der C-Fragment-TetanustoxincDNA versehen ist;
4. mit einem Retrovirus behandelt, der mit der C-Fragment-TetanustoxincDNA und der CTLA4Ig-cDNA versehen ist;
5. mit einem Retrovirus behandelt, der mit der C-Fragment-TetanustoxincDNA und der CD40-Antisense-cDNA versehen ist;
6. mit einem Retrovirus behandelt, der mit der C-Fragment-TetanustoxincDNA, der CD40-Antisense-cDNA und der CTLA4Ig-cDNA versehen ist.

Die Messungen wurden sieben Tage nach Beginn der Behandlungen durchgeführt. Der T-Zell-Proliferation-Assay wurde mit dem EZ4U-Kit (BIOMEDICA, Wien, Österreich) durchgeführt.

| Reaktion der T-Zellen in relativen Einheiten: | |
|---|---|
| zu 1.: | 0 |
| zu 2.: | 0 |
| zu 3.: | +++ |
| zu 4. | + |
| zu 5.: | ++ |
| zu 6.: | + |

### Diskussion

Der Versuch zeigt, daß die Gesamtheit der T-Zell-vermittelten Immunreaktionen gegen in diesem Falle Tetanustoxin vermindert werden kann, wenn die Monozyten nicht nur spezifisch antigene Determinaten des Tetanustoxins präsentieren, sondern zusätzlich die Bindung von B7- und/oder CD40-Molekülen an die entsprechenden Liganden verhindert wird. Hier sind sowohl Th1 als auch Th2 T-Zell-Aktivierungen vermindert worden. Die Blockade von B7 ist dabei effizienter als die von CD40.

### Ergebnisse an Tieren, in vivo

Der Versuchsansatz enthält Tests, mit Monozyten und T-Lymphozyten. Die Monozyten wurden
1. nicht genetisch verändert;
2. mit einem Retrovirus behandelt, der keine therapeutischen Gene enthält;
3. mit einem Retrovirus behandelt, der mit der OvalbuminIi-cDNA versehen ist;
4. mit einem Retrovirus behandelt, der mit der OvalbuminIi-cDNA und der CTLA4Ig-cDNA versehen ist;
5. mit einem Retrovirus behandelt, der mit der OvalbuminIi-cDNA und der CD40-Antisense-cDNA versehen ist;
6. mit einem Retrovirus behandelt, der mit der OvalbuminIi-cDNA, der CD40-Antisense-cDNA und der CTLA4Ig-cDNA versehen ist.

OvalbuminIi bedeutet, daß das Ovalbumin mit einer Signalsequenz für das MHC II-Kompartiment versehen wurde, um seine Fragmente mit größerer Effizienz an MHC II präsentieren zu lassen.

### Antikörperspiegel der gegen Ovalbumin geimpften Mäuse in relativen Einheiten:

| Zeit in Wochen | 0 | 1 | 4 | 8 |
|---|---|---|---|---|
| zu 1.: | +++++ | +++++ | +++++ | ++++ |
| zu 2.: | +++++ | +++++ | +++++ | ++++ |
| zu 3. | +++++ | +++++ | +++++++ | ++++++++ |
| zu 4.: | +++++ | +++++ | +++ | ++ |
| zu 5.: | +++++ | +++++ | ++++ | +++ |
| zu 6.: | +++++ | +++++ | +++ | ++ |

### Diskussion

Der Versuch zeigt, dass die humorale Immunantwort gegen in diesem Falle Ovalbumin vermindert werden kann, wenn die Monozyten nicht nur spezifisch antigene Determinaten des Ovalbumins präsentieren, sondern zusätzlich die Bindung von B7- und/oder CD40-Molekülen an die entsprechenden Liganden verhindert wird. Wir haben hier vor allem Th2 T-Zell-Aktivierungen beobachtet. Die Blockade von B7 ist dabei effizienter als die von CD40. Es hat sich auch gezeigt, das die Verwendung der Signalsequenz der Invarianten Kette gut geeignet ist, um den gewünschten Effekt zu erhalten. In den beiden gezeigten Beispielen wurde das System an Menschen- und Mäuseblut getestet. Es zeigte sich, dass sowohl in vitro, als auch in vivo eine Unterdrückung spezifischer Immunantworten möglich ist.

Neben der erfindungsgemäß beschriebenen Transduktion von Antigen-präsentierenden Zellen sind dem Fachmann auch andere Möglichkeiten zur Transduktion, wie die Verwendung anderer Viren oder der nicht-virale Gentransfer an sich bekannt.

### Literatur

**Daikh D., Wofsy D. and Imboden J.** (1997) The CD28-B7 constimulatory pathway and its role in autoimmune disease. J Leukoc Biol **62,** 165-62.

**Greenfield E., Nguyen K. and Kuchroo V.** (1998) CD28/B7 costimulation: a review. Crit Rev Immunol **18,** 389-418

**Janeway J.C.A. and Travers P.** (1997) Immunobiology - The immune system in health and disease. Current Biology Ltd./Garland Publishing Inc., London, San Francisco and New York

**Johnson-Leger C., Christensen J. and Klaus G.** (1998) CD28 co-stimulation stabilizes the expression of the CD40 lingand on T cells. Int Immunol **10,** 1083-91

**McAdam A., Schweitzer A. and Sharpe A.** (1998) The role of B7 costimulation in activation and differentiation of CD4+ and CD8+ T cells. Immunol Rev **165,** 231-47

**Steinman L.** (1994) Autoimmunerkrankungen. Spektrum der Wissenschaften (Spezial: Das Immunsystem) 64-73.

**Vyth-Dreese F., Dellemijn T., Majoor D. and de Jong D.** (1995) Localization in situ of the co-stimulatory molecules B7.1, B7.2, DC40 and their ligands in normal human lymphoid tissue. Eur J Immunol **25,** 3023-9

**Linsley Peter S; Ledbetter Jeffrey A; Damle Nitin K; Brady William** (Publication date: 1993-01-07) CTL4A RECEPTOR, FUSION PROTEINS CONTAINING IT AND USES THEREOF. Patent Number: WO9300431

**Bertran J. Miller, J., Yang, Y., et al., .** (1996) *Recombinant adeno-associated virus-mediated high-efficiency, transient expression of the murine cationic amino acid transporter (ecotropic retroviral receptor) permits stable transduction of human HeLa cells by ecotropic retroviral vectors. J-Virol*. 70, 6759-66.

**Boshart M., Weber F., Jahn G., Dorsch-Hasler K., Fleckenstein B. and Schaffner W.** (1985) A very strong enhancer is located upstream of an immediate early gene of human cytomegalovirus. *Cell* **41,** 521-30.

**Danos O.a.M. R.C.** (1988) *Safe and efficient generation of recombinant retroviruses with amphotropic and ecotropic host ranges. Genetics* **85,** 6460-6464.

**Fields B.N.** (1996) *Fields Virology.* Lippincot-Raven, Philadelphia

Fred **M. Ausubel R.B. Robert E. Kingston, David D. Moore, J.G. Seidman, John A. Smith,** Kevin Struhl (1999) Current Protocols in Molecular Biology. John Wiley & Sons, New York

**Hildinger M., Schilz A., Eckert H.G., Bohn W., Fehse B., Zander A., Ostertag W. and Baum C.** (1999) Bicistronic retroviral vectors for combining myeloprotection with cell-surface marking. *Gene Ther* **6,** 1222-30.

**Hunt N. Laker, C., Stocking, C., et al.** (1987) *Retroviral vectors for gene transfer into and gene expression in hematopoietic cells.* In *Advanced Research Workshop " Molecular and Cellular Aspects of Erythropoiesis",* (Rich I.N., eds) Berlin: Springer-Verlag, pp. 103-121.

**John E. Coligan A.M.K. David H. Margulies, Ethan M. Shevach, Warren Strober** (1999) Current Protocols in Immunology. John Wiley & Sons, New York

**Juan S. Bonifacino M.D. Jennifer Lippincott-Schwartz, Joe B. Harford, and Kenneth M. Yamada** (1999) Current Protocols in Cell Biology. John Wiley & Sons, New York

**Nelson J.A., Reynolds-Kohler C. and Smith** B.A. (1987) Negative and positive regulation by a short segment in the 5'-flanking region of the human cytomegalovirus major immediate-early gene. *Mol Cell Biol* **7,** 4125-9.

**Porter C.D. Collins, M., Tailor, C., et al.** (1996) *Comparison of efficiency of infection of human gene therapy target cells via four different retroviral receptors. Hum Gene Ther* **7(),** 913-9.

## Patentansprüche

1. Antigen-präsentierende Zelle, erhältlich durch Transformation einer antigen-präsentierenden Zelle mit nukleinsäurehaltigem Material, wobei das nukleinsäurehaltige Material eine erhöhte Expression eines vorher bestimmten Antigens sowie eine Suppression von Funktionen der B7-, und/oder CD40-Rezeptoren bewirkt.

2. Antigen-präsentierende Zelle nach Anspruch 1, **dadurch gekennzeichnet, dass** die antigen-präsentierende Zelle ein Monozyt, eine dendritische Zelle und/oder ein Makrophage ist.

3. Antigen-präsentierende Zelle nach Anspruch 1 und/oder 2, **dadurch gekennzeichnet, dass** die antigen-präsentierende Zelle eine erhöhte Anzahl von Homing-Rezeptoren, wie CD44, aufweist.

4. Antigen-präsentierende Zelle nach mindestens einem der Ansprüche 1 bis 3, **dadurch gekennzeichnet, dass** eine Transformation eine Suppression von Funktionen der B7-, CD40-Rezeptoren und/oder eine Erhöhung der Anzahl von Homing-Rezeptoren bewirkt.

5. Antigen-präsentierende Zelle nach mindestens einem der Ansprüche 1 bis 4 enthaltend Antisense-Nukleinsäuren zur Verhinderung der B7-und/oder CD40-Rezeptor Expression.

6. Antigen-präsentierende Zelle nach mindestens einem der Ansprüche 1 bis 4 enthaltend Nukleinsäuren, die eine Unterdrückung der Expression der B7- und/oder CD40-Rezeptoren durch Co-Suppression bewirken.

7. Antigen-präsentierende Zelle nach mindestens einem der Ansprüche 1 bis 6 enthaltend Nukleinsäuren, die eine Expression von mit B7-und/oder CD40-Rezeptoren affinen Strukturen aufweisenden Proteinen oder Peptiden bewirken.

8. Antigen-präsentierende Zelle nach Anspruch 7, wobei die Proteine, die affine Strukturen zu B7-Rezeptoren aufweisen CTLA4, CD28, Antikörper, F(ab)₂, scFv und/oder F_{ab}-Fragmente sind.

9. Antigen-präsentierende Zelle nach Anspruch 7 und/oder 8, wobei die Nukleinsäuren für eine Signalsequenz kodieren oder die Expressionsprodukte eine Signalsequenz besitzen, die den Verbleib der Expressionsprodukte im endoplasmatischen Retikulum, dem Golgi-Apparat, dem Trans-Golgi-Netzwerk oder intrazellulären Vesikeln bewirkt.

10. Antigen-präsentierende Zelle nach mindestens einem der Ansprüche 1 bis 9, **dadurch gekennzeichnet, dass** die antigen-präsentierende Zelle zur Expression von Antigenen mit Nukleinsäuren transformiert ist, die den Transport der exprimierten Antigene in MHC II-Kompartimente der Zellen ermöglicht.

11. Antigen-präsentierende Zelle nach mindestens einem der Ansprüche 1 bis 10, **dadurch gekennzeichnet, dass** die Nukleinsäuren DNA, RNA, Oligonukleotide, Polynukleotide, Ribozyme, Peptidnukleinsäuren (PNA) sind. Transformation einer antigen-präsentierenden Zelle mit nukleinsäurehaltigem Material, wobei das nukleinsäurehaltige Material eine erhöhte Expression eines vorher bestimmten Antigens sowie eine Suppression von Funktionen der B7-, und/oder CD40-Rezeptoren bewirkt.

12. Antigen-präsentierende Zelle nach Anspruch 11, **dadurch gekennzeichnet, dass** die DNA Regulationselemente wie Enhancer, Promotoren, polyA-kodierende 3'-Enden zur Transkription der DNA in RNA enthält, die RNA Regulationselemente zur Translation der RNA in Protein enthält.

13. Verfahren zur Herstellung der antigen-präsentierenden Zelle nach mindestens einem der Ansprüche 1 bis 12 durch ex vivo

14. Verfahren nach Anspruch 13, wobei eine antigen-präsentierende Zelle ex vivo durch Behandlung mit Viren, viralen Vektoren, bakteriellen Vektoren, Plasmiden, die durch viralen Gentransfer, Elektroporationstechniken, Iontophorese, ballistischen Methoden und/oder anderen Techniken zur Einschleusung von Molekülen in eine antigen-präsentierende Zelle transformiert wird.

15. Verfahren nach Anspruch 13 und/oder 14, wobei eine antigen-präsentierende Zelle oder eine antigen-präsentierende Zelle durch Behandlung mit Viren, viralen Vektoren, bakteriellen Vektoren, Plasmiden die durch viralen Gentransfer, Elektroporationstechniken, Iontophorese, ballistischen Methoden und/oder anderen Techniken zur Einschleusung von Molekülen in eine Zelle mit supprimierter Funktion co-stimulatorischer Rezeptoren transformiert, die Expression co-stimulatorischer Rezeptoren durch Verhinderung deren Expression oder die co-stimulatorischen Rezeptoren durch Reaktion mit affinen Strukturen an einer Stimulation von T-Zellen, die an die antigen-präsentierende Zelle gebunden sind, verhindert wird.

16. Verfahren nach mindestens einem der Ansprüche 13 bis 15, wobei Moleküle wie Antikörper, Proteine, Peptide, Peptidomimetica, CTLA4, CD28, CD40L und/oder Bestandteile und/oder Kombinationen dieser Moleküle, die z.B. B7-1, B7-2, CD40 binden, welche eine in Gegenwart einer Antigenpräsentation stattfindende Co-Stimulation der T-Zelle behindert, mit der antigen-präsentierenden Zelle in Kontakt gebracht werden.

17. Verfahren nach mindestens einem der Ansprüche 13 bis 16, wobei die Moleküle gemäß Anspruch 16 durch Vehikel, wie Liposomen, Hydrogele, Zyklodextrine, biologisch abbaubare Nanokapseln, bio-adhäsive Mikrokugeln und/oder durch Elektroporationstechniken, Iontophorese, ballistische Methoden und/oder andere Techniken zur Einschleusung von Molekülen in die antigen-präsentierende Zelle oder die antigen-präsentierende Zelle transferiert werden.

18. Verfahren nach mindestens einem der Ansprüche 13 bis 17, wobei Nukleinsäuren durch Viren, virale Vektoren, bakterielle Vektoren, Plasmide die durch viralen Gentransfer, Elektroporationstechniken, Iontophorese, ballistische Methoden und/oder andere Techniken zur Einschleusung von Molekülen in die antigen-präsentierende Zelle oder die antigen-präsentierende Zelle transferiert werden.

19. Arzneimittel enthaltend mindestens eine antigen-präsentierende Zelle nach mindestens einem der Ansprüche 1 bis 12.

20. Arzneimittel nach Anspruch 19, wobei mindestens eine antigen-präsentierende Zelle als Infusionslösung zur intravenösen oder intraperitonealen Applikation formuliert ist.

21. Verwendung mindestens einer antigen-präsentierenden Zelle nach mindestens einem der Ansprüche 1 bis 12 zur Herstellung eines Arzneimittels zur Behandlung von ungewollten Immunreaktionen, wie Autoimmunerkrankungen und Allergien oder gewollt hervorgerufenen Immunreaktionen, wie bei Immunisierungen.

22. Verwendung mindestens einer antigen-präsentierenden Zelle nach mindestens einem der Ansprüche 1 bis 12 zur Herstellung eines Arzneimittels zur Behandlung von Immunreaktionen gegen allologe und/oder xenologe Gewebsmerkmale.

23. Verwendung nach Anspruch 21, wobei die zu behandelnden Immunreaktionen in Verbindung mit Antigenen oder deren Gensequenzen und/oder Teilen davon stehen ausgewählt aus der Gruppe bestehend aus
- Enzymen, deren Gensequenzen und/oder Teilsequenzen, insbesondere Glutamic acid decarboxylase (GAD), Rezeptor-Typ Protein Tyrosin Phosphatase IA-2Beta, H⁺K⁺ATPase, U1RNP, Transglutaminase, Argininosuccinatelyase (ASL), Tyrosinase-related protein-2, Thyroid Peroxidase, Faktor VIII, Faktor IX;
- Rezeptoren, deren Gensequenzen und/oder Teilsequenzen, insbesondere Acetylcholinrezeptor vom Nicotintyp, Myasthenia gravis, β1-adrenerger Rezeptor, α1-adrenerger-Rezeptor, Angiotensin-2-AT1-Rezeptor, Glutamat-Rezeptor, Thyrotropin-stimulierendes Hormon (TSH)-Rezeptor, LFA-1, HLA-B27, Epididymal Protein DE, Zona Pellucida (ZP)-3 Glycoprotein, Zona Pellucida (ZP)-4 Glycoprotein, Follicle-Stimulating Hormone (FSH) Rezeptor, Sperm Immunogen SP-10 oder Sperm Protein SP-10;
- Hormone oder Botenstoffe, deren Gensequenzen und/oder Teilsequenzen, insbesondere Insulin, Thyroglobulin, Follicle-Stimulating Hormone (FSH), Prostaglandin F2 alpha, Gonadotropin-Releasing Hormone (GnRH), Oestradiol-17beta, Oestrogen, Luteinizing Hormon (LH) Rezeptor, Inhibin, Testosteron, Androgen, Chorionic Gonadotrophin (CG), Interleukine, Interferone, Cytokine, Chemokine, Bone Morphogenetic Factors, β-Interferon, Estradiol;
- Strukturproteine, deren Gensequenzen und/oder Teilsequenzen, insbesondere Myelin Basic Protein (MBP), Proteolipid protein (PLP), Myelin oligodendrocyte glycoprotein (MOG), α-Fodrin, Nicht-erythroides α
- Spectrin, Beta-Amyloid Precursor Protein (beta-APP), Typ 2 Kollagen, Sperm Plasma Membran Protein PH-20;
- Antigene, deren Gensequenzen und/oder Teilsequenzen, insbesondere CENP- A Autoantigen, Beta2GP-I, ribosomales P Protein, Ro/SSA, La/SSB, Sm/RNP, Sm, Scl-70, Jo-1, BCOADC-E2, Albumin, Glucagon, Inselzellantigene, Retinal S Ag;
- Allergene, die eine IgE-Antwort auslösen, deren Gensequenzen und/oder Teilsequenzen, insbesondere Der f 1, Der f 2, Der f 3, Der p 1, Der p 2, Der p 3, Der p 4, Der p 5, Der p 8, Eur m 1, Lep d 2, Fel d 1, Can f 1, Can f 2, Mus m 1, Rat n 1, Bla g 1, Bla g 2, Bla g 4, Bla g 5, Per a 1, Bienengift Phospholipase A₂ (PLA₂), Group V major allergen Phl p 5b von Timothy Grass Pollen, Hom s 1.

24. Verwendung nach Anspruch 22, wobei die zu behandelnden Immunreaktionen in Verbindung mit allologen und/oder xenologen Gewebsmerkmalen, deren Gensequenzen und/oder Teilsequenzen, insbesondere MHC I, MHC II, Rhesus Faktor steht.

## Claims

1. An antigen-presenting cell obtainable by transformation of an antigen-presenting cell with nucleic-acid containing material, said nucleic-acid containing material causing an enhanced expression of a predefined antigen and suppression of functions of B7 and/or CD40 receptors.

2. The antigen-presenting cell according to claim 1, **characterized in that** said antigen-presenting cell is a monocyte, a dendritic cell and/or a macrophage.

3. The antigen-presenting cell according to claim 1 and/or 2, **characterized in that** said antigen-presenting cell has an increased number of homing receptors, such as CD44.

4. The antigen-presenting cell according to at least one of claims 1 to 3, **characterized in that** a transformation causes a suppression of functions of the B7, CD40 receptors and/or an increase of the number of homing receptors.

5. The antigen-presenting cell according to at least one of claims 1 to 4, containing antisense nucleic acids for preventing the expression of B7 and/or CD40 receptors.

6. The antigen-presenting cell according to at least one of claims 1 to 4, containing nucleic acids which cause a suppression of the expression of B7 and/or CD40 receptors by co-suppression.

7. The antigen-presenting cell according to at least one of claims 1 to 6, containing nucleic acids which cause expression of proteins or peptides having structures with affinity for B7 and/or CD40 receptors.

8. The antigen-presenting cell according to claim 7, wherein said proteins having structures with affinity for B7 receptors are CTLA4, CD28, antibodies, F(ab)₂, scFv and/or F_{ab} fragments.

9. The antigen-presenting cell according to claim 7 and/or 8, wherein said nucleic acids code for a signal sequence, or the expression products have a signal sequence which causes the expression products to remain in the endoplasmic reticulum, the Golgi apparatus, the trans-Golgi network, or intracellular vesicles.

10. The antigen-presenting cell according to at least one of claims 1 to 9, **characterized in that** said antigen-presenting cell is transformed for the expression of antigens with nucleic acids which enable the transport of the expressed antigens into MHC II compartments of the cells.

11. The antigen-presenting cell according to at least one of claims 1 to 10, **characterized in that** said nucleic acids are DNA, RNA, oligonucleotides, polynucleotides, ribozymes, peptide nucleic acids (PNA).

12. The antigen-presenting cell according to claim 11, **characterized in that** said DNA has regulatory elements, such as enhancers, promoters, polyA-coding 3' termini for the transcription of the DNA into RNA which contains RNA regulatory elements for the translation of the RNA into protein.

13. A method for the preparation of the antigen-presenting cell according to at least one of claims 1 to 12 by ex vivo transformation of an antigen-presenting cell with nucleic-acid containing material, said nucleic-acid containing material causing an enhanced expression of a predefined antigen and suppression of functions of B7 and/or CD40 receptors.

14. The method according to claim 13, wherein an antigen-presenting cell is transformed ex vivo by treatment with viruses, viral vectors, bacterial vectors, plasmids, which are introduced into an antigen-presenting cell by viral gene transfer, electroporation techniques, iontophoresis, ballistic methods and/or other techniques for the introduction of molecules.

15. The method according to claim 13 and/or 14, wherein an antigen-presenting cell or an antigen-presenting cell is transformed into a cell with a suppressed function of co-stimulatory receptors by treatment with viruses, viral vectors, bacterial vectors, plasmids, which are introduced by viral gene transfer, electroporation techniques, iontophoresis, ballistic methods and/or other techniques for the introduction of molecules, the expression of co-stimulatory receptors is prevented, or the co-stimulatory receptors are prevented from stimulating T cells bound to the antigen-presenting cell by reaction with affinity structures.

16. The method according to at least one of claims 13 to 15, wherein molecules such as antibodies, proteins, peptides, peptidomimetics, CTLA4, CD28, CD40L and/or components and/or combinations of these molecules which bind, for example, B7-1, B7-2, CD40, which hinders co-stimulation of the T cell occurring in the presence of antigen-presentation, are contacted with the antigen-presenting cell.

17. The method according to at least one of claims 13 to 16, wherein the molecules as defined in claim 16 are transferred into the antigen-presenting cell or antigen-presenting cell by vehicles, such as liposomes, hydrogels, cyclodextrins, biodegradable nanocapsules, bioadhesive microspheres and/or by electroporation techniques, iontophoresis, ballistic methods and/or other techniques for the introduction of molecules.

18. The method according to at least one of claims 13 to 17, wherein nucleic acids are transferred by viruses, viral vectors, bacterial vectors, plasmids, which are introduced into the antigen-presenting cell or antigen-presenting cell by viral gene transfer, electroporation techniques, iontophoresis, ballistic methods and/or other techniques for the introduction of molecules.

19. A medicament containing at least one antigen-presenting cell according to at least one of claims 1 to 12.

20. A medicament according to claim 19, wherein at least one antigen-presenting cell is formulated as an infusion for intravenous or intraperitoneal administration.

21. Use of at least one antigen-presenting cell according to at least one of claims 1 to 12 for the preparation of a medicament for the treatment of undesirable immune reactions, such as auto-immune diseases and allergies, or deliberately induced immune reactions, as in immunizations.

22. Use of at least one antigen-presenting cell according to at least one of claims 1 to 12 for the preparation of a medicament for the treatment of immune reactions against allologous and/or xenologous tissue parameters.

23. The use according to claim 21, wherein the immune reactions to be treated are related to antigens or their gene sequences and/or parts thereof selected from the group consisting of
- enzymes, their gene sequences and/or partial sequences, especially glutamic acid decarboxylase (GAD), receptor-type protein tyrosine-phosphatase IA-2beta, H⁺K⁺ATPase, U1RNP, transglutaminase, argin-inosuccinate-lyase (ASL), tyrosinase-related protein-2, thyroid peroxidase, factor VIII, factor IX;
- receptors, their gene sequences and/or partial sequences, especially acetylcholine receptor of the nicotine type, myasthenia gravis, β1-adrenergic receptor, α1-adrenergic receptor, angiotensin-2-AT1 receptor, glutamate receptor, thyrotropin-stimulating hormone (TSH) receptor, LFA-1, HLA-B27, epididymal protein DE, zona pellucida (ZP)-3 glycoprotein, zona pellucida (ZP)-4 glycoprotein, follicle-stimulating hormone (FSH) receptor, sperm immunogen SP-10 or sperm protein SP-10;
- hormones or messengers, their gene sequences and/or partial sequences, especially insulin, thyroglobulin, follicle-stimulating hormone (FSH), prostaglandin F2 alpha, gonadotropin-releasing hormone (GnRH), estradiol-17-beta, estrogen, luteinizing hormone (LH) receptor, inhibin, testosterone, androgen, chorionic gonadotropin (CG), interleukins, interferons, cytokines, chemokines, bone morphogenetic factors, β-interferon, estradiol;
- structural proteins, their gene sequences and/or partial sequences, especially myelin basic protein (MBP), proteolipid protein (PLP), myelin oligodendrocyte glycoprotein (MOG), α-fodrin, non-erythroid α-spectrin, beta-amyloid precursor protein (beta-APP), type 2 collagen, sperm plasma membrane protein PH-20;
- antigens, their gene sequences and/or partial sequences, especially CENP-A auto-antigen, beta2GP-I, ribosomal P protein, Ro/SSA, La/SSB, Sm/RNP, Sm, Scl-70, Jo-1, BCOADC-E2, albumin, glucagon, islet cell antigens, retinal S Ag;
- allergens triggering an IgE response, their gene sequences and/or partial sequences, especially Der f 1, Der f 2, Der f 3, Der p 1, Der p 2, Der p 3, Der p 4, Der p 5, Der p 8, Eur m 1, Lep d 2, Fel d 1, Can f 1, Can f 2, Mus m 1, Rat n 1, Bla g 1, Bla g 2, Bla g 4, Bla g 5, Per a 1, bee venom phospholipase A₂ (PLA₂), group V major allergen Phl p 5b from Timothy grass pollen, Hom s 1.

24. The use according to claim 22, wherein the immune reactions to be treated are related to allologous and/or xenologous tissue parameters, their gene sequences and/or partial sequences, especially MHC I, MHC II, rhesus factor.

## Revendications

1. Cellule présentatrice d'antigènes, pouvant être obtenue par transformation d'une cellule présentatrice d'antigènes avec une matière contenant des acides nucléiques, la matière contenant des acides nucléiques provoquant une expression accrue d'un antigène préalablement déterminé ainsi qu'une suppression des fonctions des récepteurs B7 et/ou CD40.

2. Cellule présentatrice d'antigènes selon la revendication 1, **caractérisée en ce que** la cellule présentatrice d'antigènes est un monocyte, une cellule dendritique et/ou un macrophage.

3. Cellule présentatrice d'antigènes selon la revendication 1 ou 2, **caractérisée en ce que** la cellule présentatrice d'antigènes comporte un nombre accru de récepteurs de guidage tels que le CD44.

4. Cellule présentatrice d'antigènes selon au moins l'une des revendications 1 à 3, **caractérisée en ce qu'**une transformation induit une suppression des fonctions des récepteurs B7, CD40 et/ou une augmentation du nombre de récepteurs de guidage.

5. Cellule présentatrice d'antigènes selon au moins l'une des revendications 1 à 4, contenant des acides nucléiques anti-sens destinés à empêcher l'expression du récepteur B7 et/ou CD40.

6. Cellule présentatrice d'antigènes selon au moins l'une des revendications 1 à 4 contenant des acides nucléiques, qui produisent une suppression de l'expression des récepteurs B7 et/ou CD40 par co-suppression.

7. Cellule présentatrice d'antigènes selon au moins l'une des revendications 1 à 6 contenant des acides nucléiques, qui induisent une expression de protéines ou de peptides présentant des structures affines avec les récepteurs B7 et/ou CD40.

8. Cellule présentatrice d'antigènes selon la revendication 7, dans laquelle les protéines, qui présentent des structures affines avec les récepteurs B7, sont des CTLA4, des CD 28, des anticorps, des F(ab)₂, des scFv et/ou des fragments Fab.

9. Cellule présentatrice d'antigènes selon la revendication 7 ou 8, dans laquelle les acides nucléiques codent pour une séquence signal ou les produits d'expression possèdent une séquence signal, qui induit la rémanence des produits d'expression dans le réticulum endoplasmique, l'appareil de Golgi, le réseau transgolgien ou les vésicules intracellulaires.

10. Cellule présentatrice d'antigènes selon au moins l'une des revendications 1 à 9, **caractérisée en ce que** la cellule présentatrice d'antigènes est transformée pour l'expression des antigènes avec des acides nucléiques, qui rendent possible le transport des antigènes exprimés dans les compartiments du MCH Il des cellules.

11. Cellule présentatrice d'antigènes selon au moins l'une des revendications 1 à 10, **caractérisée en ce que** les acides nucléiques sont des ADN, des ARN, des oligonucléotides, des polynucléotides, des ribozymes, des acides nucléiques peptidiques (PNA).

12. Cellule présentatrice d'antigènes selon la revendication 11, **caractérisée en ce que** l'ADN contient des éléments de régulation tels que des activateurs, des promoteurs, des chaînes à extrémité 3' codant le Poly A, et l'ARN contient des éléments de régulation pour la traduction de l'ARN en protéine.

13. Procédé de préparation de la cellule présentatrice d'antigènes selon au moins l'une des revendications 1 à 12 par une transformation ex vivo d'une cellule présentatrice d'antigènes avec une matière contenant des acides nucléiques provoquant une expression accrue d'un antigène préalablement déterminé ainsi qu'une suppression des fonctions des récepteurs B7 et/ou CD40.

14. Procédé selon la revendication 13, dans lequel on transforme une cellule présentatrice d'antigènes ex vivo par traitement avec des virus, des vecteurs viraux, des vecteurs bactériens, des plasmides, par transfert de gènes viraux, des techniques d'électroporation, par ionophorèse, des techniques balistiques et/ou d'autres techniques de transfection de molécules dans une cellule présentatrice d'antigènes.

15. Procédé selon la revendication 13 et/ou 14, dans lequel on transforme une cellule présentatrice d'antigènes ou une cellule présentatrice d'antigènes par traitement avec des virus, des vecteurs viraux, des vecteurs bactériens, des plasmides, par transfert de gènes viraux, des techniques d'électroporation, ionophorèse, des techniques balistiques et/ou d'autres techniques de transfection de molécules dans une cellule présentant une fonction supprimée des récepteurs co-stimulants, l'expression des récepteurs co-stimulants étant inhibée par inhibition de leur expression ou les récepteurs co-stimulants étant inhibés par réaction avec des structures affines dans le cadre d'une stimulation des cellules T, qui sont liées à la cellule présentatrice d'antigènes.

16. Procédé selon au moins l'une des revendications 13 à 15, dans lequel on met en contact avec la cellule présentatrice d'antigènes des molécules telles que des anticorps, des protéines, des peptides, des peptidomimétiques, des CTLA4, des CD28, des CD40L et/ou des constituants et/ou des combinaisons de ces molécules, qui lient par ex. le B7-1, le B7-2, le CD40, et qui entravent une co-stimulation de la cellule T ayant lieu en présence d'une présentation d'antigène.

17. Procédé selon au moins l'une des revendications 13 à 16, dans lequel on transfère les molécules selon la revendication 16 par des véhicules tels que des liposomes, des hydrogels, des cyclodextrines, des nanocapsules biologiquement dégradables, des microbilles bio-adhésives et/ou par des techniques d'électroporation, ionophorèse, des méthodes balistiques et/ou d'autres méthodes de transfection de molécules dans la cellule présentatrice d'antigènes ou la cellule présentatrice d'antigènes.

18. Procédé selon au moins l'une des revendications 13 à 17, dans lequel on transfère les acides nucléiques par des virus, des vecteurs viraux, des vecteurs bactériens, des plasmides, par transfert de gènes viraux, des techniques d'électroporation, ionophorèse, des techniques balistiques et/ou d'autres techniques de transfection de molécules dans la cellule présentatrice d'antigènes ou la cellule présentatrice d'antigènes.

19. Médicament contenant au moins une cellule présentatrice d'antigènes selon au moins l'une des revendications 1 à 12.

20. Médicament selon la revendication 19, dans lequel au moins une cellule présentatrice d'antigènes est formulée sous forme d'une solution de perfusion destinée à une administration intraveineuse ou intrapéritonéale.

21. Utilisation d'au moins une cellule présentatrice d'antigènes selon au moins l'une des revendications 1 à 12 pour préparer un médicament destiné au traitement des réactions immunitaires non voulues, comme les maladies auto-immunes et les allergies ou des réactions immunitaires provoquées volontairement, comme dans le cas d'immunisations.

22. Utilisation d'au moins une cellule présentatrice d'antigènes selon au moins l'une des revendications 1 à 12 pour préparer un médicament destiné au traitement des réactions immunitaires contre des signes caractéristiques tissulaires allologues et/ou hétérologues.

23. Utilisation selon la revendication 21, dans laquelle les réactions immunitaires à traiter en liaison avec les antigènes ou leurs séquences génétiques et/ou des parties d'entre elles sont sélectionnées dans le groupe comportant les éléments suivants :
- les enzymes, leurs séquences génétiques et/ou leurs séquences partielles, notamment la décarboxylase de l'acide glutamique (GAD), le type de récepteur protéine tyrosine phosphatase IA-2Béta, la H⁺K⁺ATPase, le U1RNP, la transglutaminase, l'argininosuccinatelyase (ASL), la Tyrosinase-related protein-2, la péroxydase de la thyroïde, le facteur VIII, le facteur IX ;
- les récepteurs, leurs séquences génétiques et/ou leurs séquences partielles, notamment le récepteur nicotinique de l'acétylchloline, le Myasthenia gravis, le récepteur adrénergique β-1, le récepteur adrénergique α-1, le récepteur d'angiotensine-2-AT1, le récepteur du glutamate, le récepteur de l'hormone thyréotrope (TSH), le LFA-1, le HLA-B27, la protéine épédidymale DE, la glycoprotéine ZP3 de la membrane pellucide, la glycoprotéine ZP4 de la membrane pellucide, le récepteur de l'hormone folliculo-stimulante (FSH), le Sperm Immunogen SP-10 ou la Sperm Protein SP-10 ;
- les hormones ou les messagers, leurs séquences génétiques et/ou leurs séquences partielles, notamment l'insuline, la thyroglobuline, l'hormone folliculostimulante (FSH), la prostaglandine F2 alpha, l'hormone de libération des gonadotropines (GnRH), l'oestradiol-17béta, les oestrogènes, le récepteur de l'hormone lutéinisante (LH), l'inhibine, la testostérone, les androgènes, la gonadotrophine chorionique (CG), les interleukines, les interférons, les cytokines, les chimiokines, les facteurs de morphogénie des os, le β-interféron, l'estradiol ;
- les protéines structurelles, leurs séquences génétiques et/ou leurs séquences partielles, notamment la protéine basique de la myéline (MBP), la protéine du protéolipide (PLP), la glycoprotéine oligodendrocyte de la myéline (MOG), la α-fodrine, la α-spectrine non érythroïde, la protéine précurseur du béta-amyloïde (béta-APP), le collagène de type 2, la protéine de la membrane du plasma du sperme PH-20 ;
- les antigènes, leur séquences génétiques et/ou leurs séquences partielles, notamment l'autoantigène de la CENP- A, le Béta2GP-I, la protéine P ribosomale, le Ro/SSA, le La/SSB, le Sm/RNP, le Sm, le Scl-70, le Jo-1, BCOADC-E2, l'albumine, le glucagon, les antigènes de cellule des îlots de Langerhans, le Retinal S Ag ;
- les allergènes, qui déclenchent une réponse d'IgE, leurs séquences génétiques et/ou leurs séquences partielles, notamment Der f 1, Der f 2, Der f 3, Der p 1, Der p 2, Der p 3, Der p 4, Der p 5, Der p 8, Eur m 1, Lep d 2, Fel d 1, Can f 1, Can f 2, Mus m 1, Rat n 1, Bla g 1, Bla g 2, Bla g 4, Bla g 5, Per a 1, la phospholipase A₂ (PLA₂) de venin d'abeilles, l'allergène major du groupe V Phl p 5 b du Timothy Grass Pollen, le HOM s 1.

24. Utilisation selon la revendication 22, dans laquelle les réactions immunes à traiter en relation avec des signes caractéristiques tissulaires allologues et/ou hétérologues, leurs séquences génétiques et/ou séquences partielles sont apparentées notamment au CMH I, CMH II, au facteur rhésus.
